(19) Europäisches Patentamt / European Patent Office / Office européen des brevets

(11) **EP 4 028 539 B1**

(12) **EUROPEAN PATENT SPECIFICATION**

(45) Date of publication and mention of the grant of the patent:
**27.11.2024 Bulletin 2024/48**

(21) Application number: **20767575.2**

(22) Date of filing: **10.09.2020**

(51) International Patent Classification (IPC):
*C12Q 1/04* $^{(2006.01)}$     *C12M 1/34* $^{(2006.01)}$
*C12Q 1/18* $^{(2006.01)}$

(52) Cooperative Patent Classification (CPC):
**C12Q 1/04; C12Q 1/18**

(86) International application number:
**PCT/EP2020/075311**

(87) International publication number:
**WO 2021/048270 (18.03.2021 Gazette 2021/11)**

(54) **MEASURING THE ANTIMICROBIAL SUSCEPTIBILITY OF MICROBES**

MESSUNG DER ANTIMIKROBIELLEN EMPFINDLICHKEIT VON MIKROBEN

MESURER LA SENSIBILITÉ ANTIMICROBIENNE DES MICROBES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **10.09.2019 GB 201913035**

(43) Date of publication of application:
**20.07.2022 Bulletin 2022/29**

(73) Proprietor: **University of Strathclyde**
**Glasgow G1 1XQ (GB)**

(72) Inventors:
- **CORRIGAN, Damion, K**
  **Glasgow G1 1XQ (GB)**
- **HOSKISSON, Paul**
  **Glasgow G1 1XQ (GB)**

(74) Representative: **Marks & Clerk LLP**
**15 Fetter Lane**
**London EC4A 1BW (GB)**

(56) References cited:
**WO-A1-80/02849**

- **DOMINGO-ROCA R ET AL.: "Rapid assessment of antibiotic susceptibility using a fully 3D-printed impedance based biosensor", BIOSENSORS AND BIOELECTRONICS, 2023**

- **GEMÜNDE ANDRÉ ET AL: "Redox Mediators in Microbial Electrochemical Systems", CHEMELECTROCHEM, vol. 9, no. 13, 13 June 2022 (2022-06-13), Chichester, XP093057537, ISSN: 2196-0216, Retrieved from the Internet <URL:https://onlinelibrary.wiley.com/doi/full-xml/10.1002/celc.202200216> DOI: 10.1002/celc.202200216**

- **WEBSTER T A ET AL: "Monitoring Pseudomonas aeruginosa in culture plates using embedded electrochemical sensors", 2014 40TH ANNUAL NORTHEAST BIOENGINEERING CONFERENCE (NEBEC), IEEE, 25 April 2014 (2014-04-25), pages 1 - 2, XP032692899, DOI: 10.1109/NEBEC.2014.6972976**

- **BADALYAN GRIGORI ET AL: "Novel sensor platform for rapid detection and quantification of coliforms on food contact surfaces", JOURNAL OF MICROBIOLOGICAL METHODS, ELSEVIER, AMSTERDAM, NL, vol. 153, 19 September 2018 (2018-09-19), pages 74 - 83, XP085500789, ISSN: 0167-7012, DOI: 10.1016/J.MIMET.2018.09.009**

- **JUSTIN D. BESANT ET AL: "Rapid electrochemical phenotypic profiling of antibiotic-resistant bacteria", LAB ON A CHIP, vol. 15, 1 January 2015 (2015-01-01), pages 2799 - 2807, XP055407704, ISSN: 1473-0197, DOI: 10.1039/C5LC00375J**

- **STUART HANNAH ET AL: "Rapid antibiotic susceptibility testing using low-cost, commercially available screen-printed electrodes", BIOSENSORS AND BIOELECTRONICS, vol. 145, 1 December 2019 (2019-12-01), AMSTERDAM, NL, pages 111696, XP055652594, ISSN: 0956-5663, DOI: 10.1016/j.bios.2019.111696**

**Description**

## FIELD OF THE INVENTION

The present invention concerns a device suitable for measuring the antimicrobial susceptibility of microbes, a system comprising the device, and a method of measuring antimicrobial susceptibility of microbes using the system.

## BACKGROUND OF THE INVENTION

The United Nations (UN) and the World Health Organisation (WHO) both cite AMR as an issue of worldwide concern and one of its greatest public health problems (Food and Agriculture Organization of the United Nations; Antimicrobial Resistance Policy Review and Development Framework (2018); and World Health Organization Global Action Plan on Antimicrobial Resistance (2015)). The National Institute for Health and Care Excellence (NICE) reports AMR as an issue requiring urgent, coordinated international action (NICE impact antimicrobial resistance (2018)). In recent years, approaches to tackling AMR such as the discovery and development of new antimicrobial drugs to both prevent and treat bacterial infections has stalled and the current drug pipeline is struggling to deliver (Todd, A., Worsley, A., Anderson, R. J., Groundwater, P. W., 2009. The Pharmaceutical Journal. 283, 359-360). However, the impact of overuse and the misprescribing of antibiotics across the healthcare and agricultural sectors has resulted in the development and spread of AMR (Davies, J., Davies, D., 2010. Microbio and Mol. Bio. Rev. 74, 417-433). Therefore, a One-Health approach (McEwen, S. A., Collignon, P. J., 2018. Microbiol Spectr. 6) to tackling AMR is required including the development of rapid and reliable diagnostic tests to ascertain the nature of infection (bacterial or non-bacterial), ensure effective use of antimicrobials and reduce the rate of development of resistance (Department of Health & Social Care. The UK's vision for AMR by 2040 and five-year national action plan, 2019).

Current gold standards for identification of pathogens are typically based upon bacterial culture on agar plates, using classical microbiological techniques (Gilligan, P. H., 2013. The Prokaryotes, Human Microbiology, 3rd ed. Springer Reference, Heidelberg, New York, Dordrecht, London). Such methods are often not fast enough for modern demands, with processing times typically being 1-2 days. Therefore, these methods are not suitable for rapid screening at the point of care. More recent technologies, such as the polymerase chain reaction (PCR) can quickly identify bacterial species. However, the technique requires complex sample processing and is often expensive to implement (Clarridge, J. E., 2004. Diseases. Clin. Microbiol. Rev. 17, 840-862). Therefore, new technologies that can quickly assess the effectiveness of antibiotics for a range of clinical samples, with minimal complexity of sample handling, and can identify which antibiotic to prescribe are needed to help mitigate the spread of antimicrobial resistance.

Electrochemical sensors offer label-free detection, low-cost production, integration with other technologies including microfluidics (Nie, Z., Nijhuis, C. A., Gong, J., Chen, X., Kumachev, A., Martinez, A. W., Narovlyansky, M., Whitesides, G. M., 2010. Lab Chip. 10, 477-483) and can be integrated with simple electronics to provide real-time data and signal readout (Wang, W., Huang, H. Y., Chen, S. C., Ho, K. C., Lin, C. Y., Chou, T. C., Hu, C. H., Wang, W. F., Wu, C. F., Luo, C. H., 2011. Sensors. 11, 8593-8610). One electrochemical technique suitable for low-cost integration and capable of real-time data capture is electrochemical impedance spectroscopy (EIS). With EIS, the impedance of the electrode-electrolyte interface is studied across a range of frequencies to establish information regarding the interface, its electron transfer properties and surrounding diffusional behaviour. Changes in impedance can be reflective of changes in bacterial growth as a function of time (Ward, A. C., Hannah, A. J., Kendrick, S. L., Tucker, N. P., MacGregor, G., Connolly, P., 2018. Biosensors and Bioelectronics. 110, 65-70; Brosel-Oliu, S., Mergel, O., Uria, N., Abramova, N., van Rijn, P., Bratov, A., 2019. Lab. Chip. 19, 1436-1447). Previously, several microorganisms have been identified using EIS such as *S. aureus* (Ward et al., supra), *Escherichia coli* (Settu, K., Liu, J. T., Chen, C. J., Tsai, J. Z., Chang, S. J., 2013. Conf. Proc. IEEE Eng. Med. Biol. Soc. 2013, 1712-1715) and *Pseudomonas aeruginosa* (Ward, A. C., Connolly, P., Tucker, N. P., 2014. PLOS One. 9, 91732).

Another electrochemical measurement technique of interest is differential pulse voltammetry (DPV). DPV is an example of a voltammetric technique, whereby a series of regular voltage pulses are superimposed upon a potential linear sweep or staircase waveform. It is a quick method, which eliminates the non-Faradaic charging current and can be used to sensitively investigate electron transfer to and from an electrode surface (Batchelor-McAuley, C., Katelhon, E., Barnes, E. O., Compton, R. G., Laborda, E., Molina, A., 2015. ChemistryOpen. 4, 224-260).

Electrochemical systems have found widespread use as biological sensors (Jiang, D., Ge, P., Wang, L., Jiang, H., Yang, M., Yuan, L., Ge, Q., Fang, W., Ju, X., 2019.

Biosensors and Bioelectronics. 130, 299-306; Russell, C., Ward, A. C., Vezza, V., Hoskisson, P., Alcorn, D., Steenson, D. P., Corrigan, D. K., 2019. Biosensors and Bioelectronics. 126, 806-814).

Webster et al., in "Monitoring Pseudomonas aeruginosa in culture plates using embedded electrochemical sensors", 40th Annual NorthEast Bioengineering Conference (NEBEC), IEEE, 2014, 1-2, describe the fabrication of a disposable electrochemical assay embedded in solid growth media agar. The selective detection of *Pseudomonas*

*aeruginosa* is reportedly achieved by applying a unique set of potentials and measuring current change using electrochemical sensors.

Badalyan et al., in "Novel sensor platform for rapid detection and quantification of coliforms on food contact surfaces", Journal of Microbiological Methods, 153, 2018, 74-83, describe a sensor platform based on screen printed carbon electrode coated by graphene modified polyacrylamide gel (GR/PAAGC) for sampling, detection and enumeration of coliform bacteria on food contact surfaces.

Besant, Sargent and Kelley, in "Rapid electrochemical phenotypic profiling of antibiotic-resistant bacteria", Lab Chip, 2015, 15, 2799-2807, describe electrochemical phenotyping that reportedly achieves rapid readout of the antibiotic susceptibility profile of a bacterial infection within one hour. The approach involves the monitoring of electrochemical reduction of a redox-active molecule, which reports on levels of metabolically-active bacteria.

The present invention provides an alternative device for use in the measurement of antimicrobial susceptibility of microbes.

## SUMMARY OF THE INVENTION

It has been found that the device of the first aspect of the invention and the system comprising the device of the first aspect of the invention are surprisingly effective in measuring microbial growth when microbes are contacted with the device. Microbial growth in the presence of antimicrobials indicates a level of antimicrobial resistance and a low antimicrobial susceptibility of the microbe. Thus, contacting the device of the invention with at least one antimicrobial and a microbe, and measuring microbial growth gives an effective indication of the antimicrobial susceptibility of the microbe. The device and system of the invention are effective in determining the antimicrobial susceptibility of microbes at low-cost and with a high sensitivity for a range of clinically-relevant pathogens.

The skilled person is aware that any reference to an aspect of the invention includes every embodiment of that aspect. For example, any reference to the first aspect of the invention includes the first aspect and all embodiments of the first aspect.

Viewed from a first aspect, the present invention provides a method of measuring the antimicrobial susceptibility of microbes comprising:

(i) contacting a device or system with at least one antimicrobial or candidate antimicrobial and a sample suspected of comprising or known to comprise microbes, wherein the device comprises:

(a) an electrode system comprising two or more electrodes; and
(b) a first substance in contact with the two or more electrodes wherein the first substance is in the form of a gel, foam or solid, which is suitable for electrical conductance and which is capable of supporting microbial growth and wherein the first substance comprises a redox mediator selected from a transition metal salt;

and the system comprises:

(a) one or more of the devices; and
(b) a potentiostat,

wherein the electrodes of the electrode system are electronically connected to the potentiostat;

(ii) applying an electrical stimulus to the electrodes at least at a first and second time point; and
(iii) sensing an electrical response from the electrodes at the at least first and second time points;
wherein a difference in electrical response between the at least first and second time points is indicative of microbial growth.

In one embodiment, the method comprises measuring microbial growth in the presence of at least one antimicrobial agent or candidate antimicrobial and further measuring microbial growth in the absence of an antimicrobial agent or candidate antimicrobial. Any difference in microbial growth measurement is an indication of antimicrobial susceptibility of the microbes.

Viewed from a second aspect, there is provided a device suitable for measuring antimicrobial activity of microbes, as defined in the first aspect, comprising:

(i) an electrode system comprising two or more electrodes;
(ii) a first substance in contact with the two or more electrodes wherein the first substance is in the form of a gel, foam or solid, which is suitable for electrical conductance and which is capable of supporting microbial growth; and

at least one antimicrobial or candidate antimicrobial in contact with the first substance wherein the at least one antimicrobial is any one selected from the group consisting of β-lactam antibiotics, macrolides, lincosamides, aminoglycosides, tetracyclines, vancomycin, sulphonamides, fluoroquinolones, chloramphenicol, nitrofurantoin and isoniazid.

The first substance may be designed to ascertain whether or not a microorganism is sensitive to said one or more antimicrobial agents.

The device of the second aspect may be used for measuring antimicrobial susceptibility of microbes.

Viewed from a third aspect, the invention provides a system suitable for measuring antimicrobial susceptibility of microbes, as defined in the first aspect, comprising:

   (i) one or more devices of the first aspect; and
   (ii) a potentiostat

wherein the electrodes of the electrode system are electronically connected to the potentiostat and at least one of the one or more devices comprises at least one antimicrobial or antimicrobial candidate in contact with the first substance wherein the at least one antimicrobial is any one selected from the group consisting of β-lactam antibiotics, macrolides, lincosamides, aminoglycosides, tetracyclines, vancomycin, sulphonamides, fluoroquinolones, chloramphenicol, nitrofurantoin and isoniazid.

In an embodiment of the third aspect, the one or more devices may be used to test the susceptibility of a microorganism to one or more antimicrobial agents. Therefore, the one or more devices may comprise different antimicrobial agents.

The system of the third aspect may be used for measuring antimicrobial susceptibility of microbes.

Viewed from a fourth aspect, the invention provides a kit-of-parts comprising:

   (i) at least one device or system of any of the previous aspects and/or embodiments; and
   (ii) at least one antimicrobial agent selected from the group consisting of β-lactam antibiotics, macrolides, lincosamides, aminoglycosides, tetracyclines, vancomycin, sulphonamides, fluoroquinolones, chloramphenicol, nitrofurantoin and isoniazid.

## LIST OF FIGURES

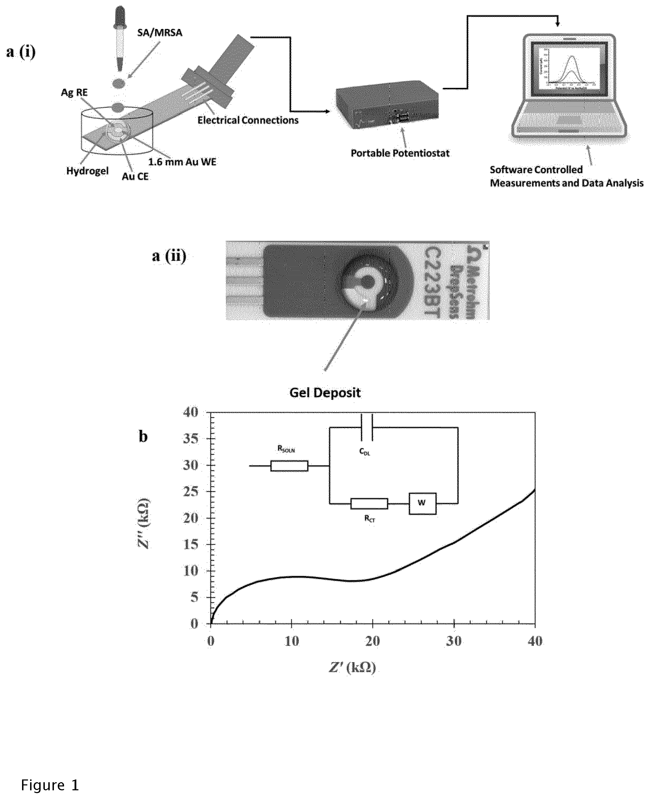

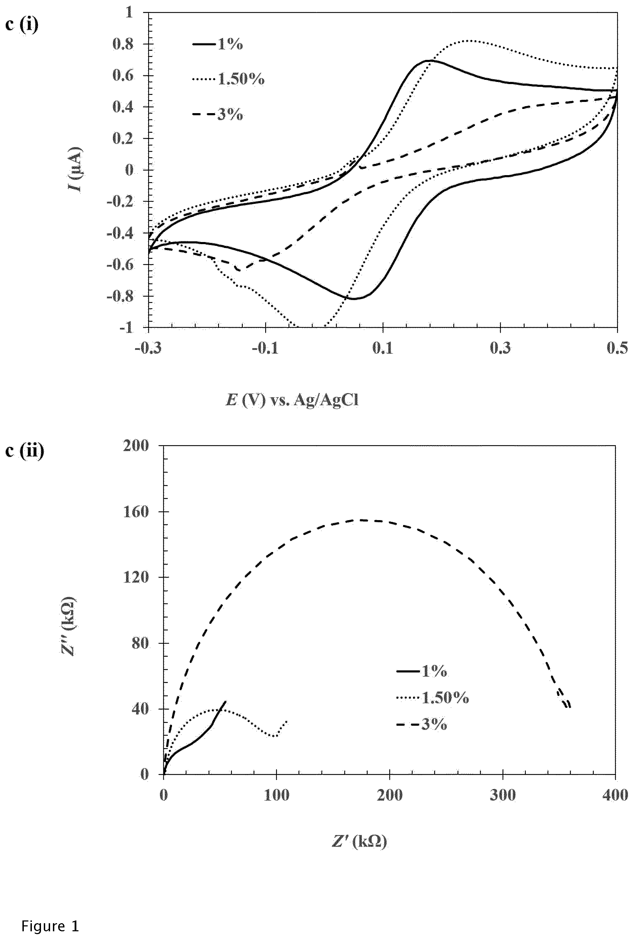

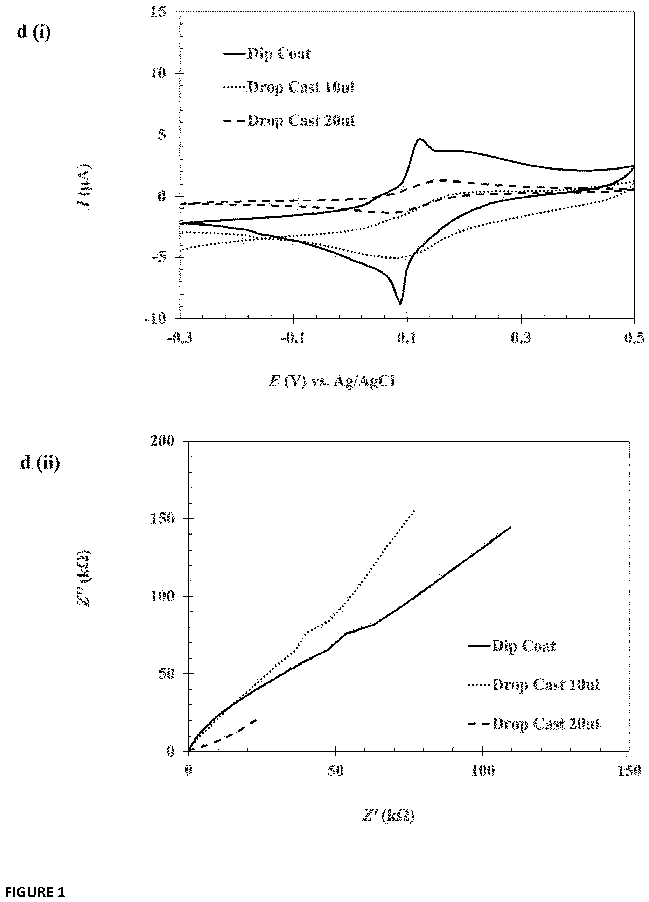

Figure 1: (a) (i) experimental setup featuring Au DropSens electrode modified with gel deposit containing agarose, LB and FF-C and some with antibiotic; schematic depicts bacteria being pipetted onto electrode, and electrochemical measurements being performed using a potentiostat; (a) (ii) electrode surface modified with agarose gel deposit; (b) typical electrochemical impedance spectroscopy (EIS) measurement; inset shows Randles' equivalent circuit model used to fit EIS data; (c) CV (i) and EIS (ii) measurements on different concentrations of agarose gel containing F-F + KCl; (d) CV (i) and EIS (ii) measurements comparing gel deposition techniques.

Figure 2: (a) CV measurements of a range of antibiotics, diluted to typical working concentrations; (b) CV measurements of 1 wt% agarose gel containing amoxicillin, with and without FF-C; (c) CV measurements of 1 wt% LB agarose gel with and without FF-C; (d) CV measurements of 20 μL and 50 μL of 1 wt% agarose gel containing FF-C.

Figure 3: bacterial growth curves of sensitive *S. aureus* (a) and MRSA (b) strains on gels infused with and without amoxicillin (8 μg/ml for *S. aureus,* and 50 μg/ml for MRSA) and baseline (no bacteria); (i) Z at 100 kHz and (ii) Z at 100 kHz - baseline curve.

Figure 4: *S. aureus* grown on streak plates on gels comprising 1 wt% agarose and LB. Gel components: (a) (i) agarose + LB, (a) (ii) agarose + LB + amoxicillin, (b) (i) agarose + LB + FF-C and (b) (ii) agarose + LB + FF-C + amoxicillin.

Figure 5: (a) *S. aureus* grown on streak plates on gels comprising 1 wt% agarose + LB + FF-C with (i) no amoxicillin and (ii) with amoxicillin. (b) MRSA grown on streak plates on gels comprising 1 wt% agarose + LB + FF-C with (i) no amoxicillin and (ii) with amoxicillin.

Figure 6: bacterial growth curves of sensitive *S. aureus* (a) and MRSA (b) strains on gels infused with and without oxacillin (Oxa) (8 μg/ml) and baseline (no bacteria); (i) Z at 100 kHz and (ii) Z at 100 kHz - baseline curve.

Figure 7: Bacterial growth curves of MRSA on gels infused with and without amoxicillin (Amox) (8 μg/ml and 50 μg/ml) and baseline (no bacteria). (i) $I_{pk}$ and (ii) $I_{pk}$ - baseline curve.

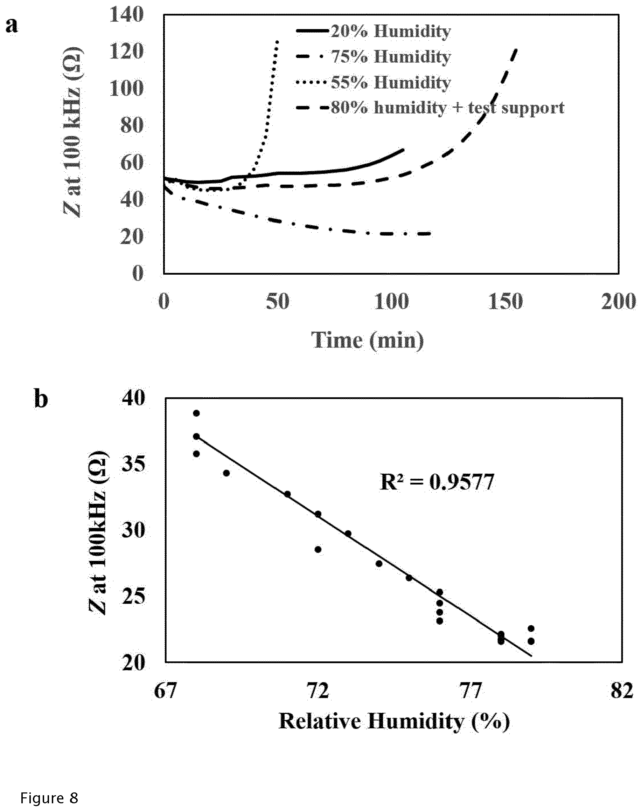

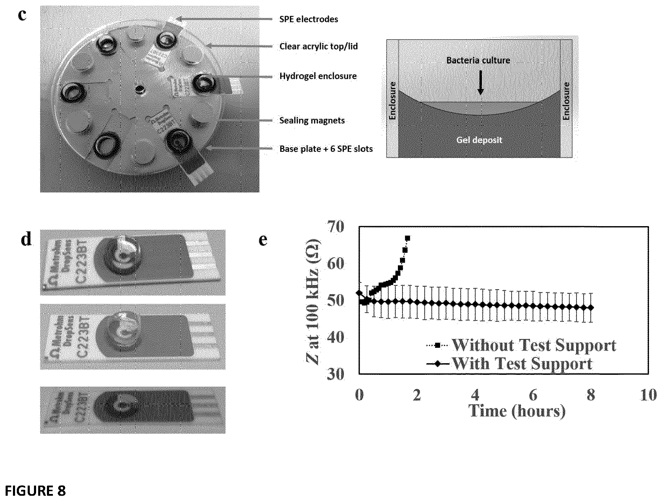

Figure 8: (left column) The effect of environmental humidity level on gel electrochemical impedance at 100 kHz. a) Summary of all experiments performed showing that, in all cases except the 75% test, the hydrogel began drying out after a certain amount of time had elapsed, indicated by a sharp increase in impedance. b) 75% humidity measurement indicating a linear dependence ($R^2$ = 0.95) between hydrogel impedance and environmental humidity level (after allowing 10 minutes for humidity stabilisation) c) Test support structure created to maintain gel integrity. Image of test support showing 3 × SPEs (left) and schematic of test support hydrogel enclosure showing gel deposit and bacteria culture (right) (d) Photograph of Au DropSens electrode modified with gel-deposit before measurement (above), after 8h baseline measurement with the test support (middle) and 'collapsed' form following 75% humidity measurement without a test support (below) e) Electrochemical baseline data with gel-only comparing Z at 100 kHz over time with and without test support (n=3 SPEs).

Figure 9: a) Z at 100 kHz % change of growth curves for gels with and without streptomycin. b) Growth curves of phase angle at 100 kHz % change for gels with and without streptomycin.

## DETAILED DESCRIPTION OF THE INVENTION

The device of the invention and the system comprising the device are surprisingly effective in measuring microbial growth and determining the antimicrobial susceptibility of microbes at low-cost for a range of clinically-relevant microbe strains. In addition, the device and system may be able to detect different classes, species or sub-species of microbes. For example, the device and system may be able to detect whether a bacterium is Gram negative or Gram positive. The device and system are now described in detail.

In the discussion that follows, reference is made to a number of terms, which have the meanings provided below, unless a context indicates to the contrary. The nomenclature used herein for defining compounds, in particular the compounds according to the invention, is in general based on the rules of the IUPAC organisation for chemical compounds, specifically the "IUPAC Compendium of Chemical Terminology (Gold Book)".

The term "comprising" or variants thereof will be understood to imply the inclusion of a stated element, integer or step, or group of elements, integers or steps, but not the exclusion of any other element, integer or step, or group of elements, integers or steps.

The term "consisting" or variants thereof will be understood to imply the inclusion of a stated element, integer or step, or group of elements, integers or steps, and the exclusion of any other element, integer or step or group of elements, integers or steps.

The "antimicrobial susceptibility of microbes" defines the likelihood that microbes will be affected by antimicrobials. "Microbes" refers to microorganisms that are generally single-celled organisms but may be multicellular. Often, microbes cause disease. "Antimicrobials" refers to compounds that inhibit the growth of, or destroy, microbes. Antimicrobials may be β-lactam antibiotics (e.g. penicillins and cephalosporins), macrolides (also known as erythromycins), lincosamides (e.g. lincomycin), aminoglycosides (e.g. gentamicin), tetracyclines (e.g. tetracycline), polypeptides (e.g. vancomycin), sulphonamides (e.g. sulfadiazine), fluoroquinolones (e.g. enrofloxacin) and others including chloramphenicol, nitrofurantoin and isoniazid.

Microbial growth occurs when the rate of microbial cell division is greater than cell death, i.e. the population of microbes increases. The growth of microbes that are susceptible to an antimicrobial will be reduced on exposure of the microbe to the antimicrobial, relevant to the growth of the microbial in the absence of the antimicrobial. When the antimicrobial is a microstatic, it halts microbial growth, thereby preventing a population of microbes from increasing in size. Alternatively, microbes that are susceptible to an antimicrobial may be destroyed by the antimicrobial, thereby shrinking the population of microbes. Antimicrobials that shrink microbe populations are known as microbicides.

The microbes may be bacteria or fungi. Thus, the device may be suitable for measuring the antibiotic susceptibility of bacteria or the antifungal susceptibility of fungi. "Bacteria" refers to microscopic, single-celled organisms that comprise cell walls but lack organelles and an organised nucleus. "Antibiotic" refers to a compound that inhibits the growth of, or destroys, bacteria. When used herein, "fungi" refers to microscopic unicellular or multicellular heterotrophic eukaryotes that contain chitin in their cell walls. Fungi include yeasts (e.g., Cryptococcus, candida) and moulds (e.g. tinea, Aspergillus) and are preferably yeasts, i.e. unicellular fungi (e.g. Candida auris).

In one embodiment, the microbes are bacteria, thus the device or system of the invention may be suitable for measuring the antibacterial susceptibility of bacteria and the first substance of the device or system is suitable of supporting bacterial growth. The growth of bacteria that are susceptible to an antibiotic will be reduced on exposure of the bacteria to the antibiotic, relevant to the growth of the bacteria in the absence of the antibiotic. When the antibiotic is a bacteriostatic, it halts bacterial growth, thereby preventing a bacterial colony from increasing in size. Alternatively, bacteria that are susceptible to an antibiotic may be destroyed by the antibiotic, thereby shrinking the bacterial colony. Antibiotics that shrink microbe populations are known as bacteriocides. The device or system of the invention may be suitable for distinguishing bacteriostatic antibiotics from bacteriocidal antibiotics with respect to a particular microbial.

The growth of different microbe species and microbe strains may be inhibited by antimicrobials to differing degrees: antimicrobial susceptibility to the same antimicrobial may range across different species and/or strains. Alternatively, a species or strain may not be affected by an antimicrobial, thereby indicating that the microbes are not susceptible to that antimicrobial.

The term "microbe strain" is defined herein to refer to a genetic variant of a species, i.e. a sub-class of a species. For example, a strain of *Staphylococcus aureus* may be a specific genetic variant of the *Staphylococcus aureus* species, which arises owing to genetic alteration.

Microbe growth occurs when the rate of microbe reproduction is greater than microbe death, i.e. the population of microbes increases. Fungi may reproduce via binary cell division, budding of cells, asexual spore formation or fragmentation of hyphae. Bacteria reproduce via cell division. During cell division, a cell divides into two daughter cells via binary fission. The two daughter cells may or may not be genetically identical to the original cell, depending on the occurrence of genetic mutations. In the absence of antimicrobials, and in the presence of nutrients necessary for microbe survival, microbe growth may be exponential.

As described, the first substance of the device in accordance with the present invention is suitable for electrical conductance and supporting microbial growth. In one embodiment, the first substance adheres (i.e. sticks) to the electrodes. By "adheres" is meant that the first substance remains in contact with the electrodes on reasonable movement (e.g. displacement or rotation of the electrodes). This means that the device can be pre-formed and/or transported prior to use, without the first substance losing contact with the electrodes. As mentioned above, the first substance is a gel. "Gel" is used herein to refer to liquid particles dispersed in a solid medium. Gels differ from liquids in that they exhibit no flow when in a steady-state, i.e. they do not flow unless disturbed. "Foam" is used herein to refer to gas pockets trapped in a liquid or solid. Sometimes, the foam is a gas trapped within a solid. In one embodiment, the first substance comprises a hydrogel.

Hydrogels are increasingly finding use within biosensor applications ranging from wound dressings with controlled release of antibiotics (Tavakoli, J., Tang, Y., 2017. Mater. Sci. Eng. C. Mater. Biol. Appl. 77, 318-325), cell stimulators for implantable bioelectronics (Han, L., Lu, X., Wang, M., Gan, D., Deng, W., Wang, K., Fang, L., Liu, K., Chan, C. W., Tang, Y., Weng, L. T., Yuan, H., 2017. Small. 13) to super capacitor electrode systems (Chen, S., Duan, J., Tang, Y., Zhang Qiao, S., 2013. Chemistry. 19, 7118-7124), amongst others. Hydrogels are macromolecular polymer gels, made up of networks of crosslinked polymer chains. Hydrogels feature many attractive advantages for biosensor applications including their inertness to biological processes, lack of degradation, permeability to metabolites, biocompatibility, ability to withstand high temperatures and their lack of absorption by the body (Michalek, J., Hobzova, R., Pradny, M., Duskova, M., 2010. Biomedical Applications of Hydrogels Handbook., 303-316). Sometimes, the hydrogel of the first substance comprises a polysaccharide. If present, the amount of polysaccharide sometimes ranges from about 0.5 to about 5 wt%, such as about 0.5 to about 3 wt%, or about 1 wt%. Sometimes, the polysaccharide comprises repeat units of a disaccharide. The disaccharide may comprise D-galactose and/or 3,6-anhydro-L-galactopyranose. Sometimes, the disaccharide comprises D-galactose and 3,6-anhydro-L-galactopyranose, i.e. the disaccharide is agarobiose. The hydrogel may comprise Agarose, which is suitable for use as a hydrogel owing to its UV transparency and low toxicity (Science Direct, 2019).

Agarose is commonly extracted from red seaweed or red algae (Jeppsson, J. O., Laurell, C. B., Franzen, B., 1979. Clin. Chem. 25, 629-638), and is frequently used in molecular biology, particularly in electrophoresis, to separate large molecules such as DNA. It is one of the two principle components of agar, and may be purified from agar through the removal of agaropectin. Agarose is a suitable material for preparing gels since it forms a network containing pores, the pore size being dependent upon the agarose concentration used. The porous nature of such gels and their ease of production makes them a suitable method for monitoring bacterial growth over time, since agarose gels can be filled with the relevant antibiotics/nutrients required to inhibit or promote bacterial growth.

Agarose performs best in gel form at relatively low concentrations in solution, for example, 1 wt% gels are commonly used to provide good separation and resolution of large DNA fragments during electrophoresis (Sabath, L. D., Garner, C., Wilcox, C., Finland, M., 1976. Antimicrobial Agents and Chemotherapy. 9, 962-969).

The first substance may comprise a growth medium containing the nutrients required to support microbial growth, e.g. a carbon source. When the microbes are fungi, a fungal medium is often used comprising, for example, dextrose and soyabean products. When the microbes are bacteria, Lysogeny Broth (LB) medium, also known as Miller LB, may be used. This is a nutritionally rich medium commonly used for bacterial growth, which comprises sodium chloride, tryptone, yeast extract and deionised water. The tryptone provides peptides and casein peptones, whilst the yeast extract provides vitamins and trace elements. LB is well known in the art, and is commercially available from, for example, Sigma Aldrich. Other suitable bacterial/microbial growth media are known to the skilled reader and may be used in the invention (see, for example J. Overmann et al., Annu. Rev. Microbiol., 71, 711-730).

The first substance of the invention is suitable for electrical conductance. In one embodiment, the first substance comprises an electrolyte. Electrolytes are substances that, when dissolved in polar solvents (for example, water), produce an electrically conducting solution. Electrolytes are salts that, on dissolution, separate into their constituent cations and anions. In the presence of an electrode system, and on the application of an electric potential, the cations of the electrolyte are attracted to the electrode acting as the anode, i.e. that with a surplus of electrons, and the anions of the electrolyte are attracted to the electrode acting as the cathode, i.e. that with a deficit of electrons. The resulting movement of ions gives rise to a current.

Suitable electrolytes of the first substance comprise a metal cation (such as an alkali or alkaline earth metal cation) and a counterion. Sometimes, the counterion is selected from any one or a combination of the group consisting of halide, sulfate, carbonate, bicarbonate, phosphate, nitrate, citrate, gluconate, acetate, oxide, lactate, glubionate, aspartate and picolinate. Often, the counterion is selected from any one or a combination of the group consisting of halide, sulfate, carbonate, bicarbonate, phosphate and nitrate. Typically, the counterion is a halide, preferably chloride.

Often, the metal cation of the electrolyte of the first substance is selected from any one or a combination of the group consisting of potassium, sodium, magnesium, calcium, zinc and chromium. Typically, the metal cation is selected from any one or a combination of the group consisting of potassium, sodium, magnesium and calcium, preferably potassium. Most commonly, the electrolyte of the first substance is potassium chloride.

The skilled reader is aware that growth media typically contain electrolytes, such as sodium chloride or potassium chloride, thus in one embodiment the growth medium of the first substance comprises one or more suitable electrolytes and a further electrolyte may not be required.

The first substance of the invention comprises a redox mediator selected from a transition metal salt. Redox mediators are chemical compounds which act as electron shuttles between oxidising species and reducing species. Often, the transition metal of the transition metal salt is selected from any one or a combination of the group consisting of iron, iridium, ruthenium, chromium, vanadium, cerium, cobalt, osmium and manganese. Commonly, the transition metal is not a combination of metals. Typically, the transition metal is selected from any one of the group consisting of iron, iridium or ruthenium. When the transition metal iron is iron, it is often in an oxidation state of III or II; when the transition metal iron is iridium, it is often in an oxidation state of III or IV, when the transition metal iron is ruthenium, it is often in an oxidation state of II or III. Often, the redox mediator is selected from any one of the group consisting of iron hexacyanide, iridium chloride, ruthenium hexamine chloride and ferrocene. Often, the redox mediator is $[Fe^{III}(CN)_6]^{3-}/[Fe^{II}(CN)_6]^{4-}$.

The concentration of redox mediator in the first substance may range from about 0.02 to about 10 mM, such as about 0.5 to about 10 mM, sometimes from about 0.7 to about 5 mM, such as about 1 to about 2 mM.

According to particular embodiments, the first substance consists essentially of a gel, a growth medium, an electrolyte, and a redox mediator. When the growth medium comprises an electrolyte, an additional electrolyte may not be present, i.e. the first substance may consist essentially of a gel, a growth medium and a redox mediator. Use of the term "consists essentially of" is meant, for example, that the presence of additional components within the first substance is permitted, provided that the amounts of such additional components do not render the first substance incapable of supporting microbial growth or conducting electricity. Thus, it will be understood that the presence of any components that allow the first substance to support microbial growth or conduct electricity is included.

In a further embodiment, the first substance consists of a gel, a growth medium, an electrolyte, and a redox mediator. Such embodiments include residual solvents which remain in the first substance following its formation.

The first substance is in contact with the electrode system. Any method of contact is suitable, but the first substance is typically deposited onto the electrode system by dip-coating, drop-casting or printing. For example, the first substance may be deposited onto the electrode system by dip-coating or drop-casting. When working with hydrogels, the deposition method is an important factor to consider because it affects reproducibility and uniformity of gel deposits from run-to-run.

Dip-coating comprises five stages:

(i) immersion, where the electrode system is immersed in a solution of the first substance at a constant speed;
(ii) start-up, where the electrode system has been immersed in the solution of the first substance for a specific time and begins to be displaced out of the solution at a constant speed;
(iii) deposition, where a thin layer of the solution of the first substance deposits itself on the electrode system as the electrode system is displaced out of the solution;
(iv) drainage, where excess liquid drains from the surface of the electrode system; and
(v) evaporation, where the solvent (typically water) evaporates from the solution of the first substance, forming a thin layer of first substance.

The speed of displacement of the electrode system from the solution of the first substance determines the thickness of the coating (a faster speed gives a thicker coating). Dip-coating is a useful method to produce a thin gel layer (when using slower speeds of displacement) on the electrode surface; however, it requires a large volume of material, making it relatively expensive.

Conversely, drop-casting is a simpler method which uses the exact volume of material required, reducing the costs and waste involved. Drop casting comprises dropping a solution of the first substance onto the electrode system and evaporating the solvent (typically water). A simple method of drop-casting is to pipette gels using a specific volume. Preferably, the first substance is contacted with the electrode system by drop-casting.

In certain embodiments, the device/system is in a controlled environment, by which is meant that it is stored and/or used in suitable conditions. In order to avoid contamination, for example with unwanted microbes, the device/system may be stored and/or used in a controlled environment. For example, in one embodiment the device/system is sealed in order to prevent contamination from the surrounding atmosphere. The seal may be reusable and may be part of the device/system. Alternatively, the seal may be disposable. Advantageously, sealing the device/system prevents the gel from drying out and lengthens the shelf-life of the device/system.

In some embodiments, the first substance is sealed from the atmosphere. Such sealing may increase retention of the integrity of the first substance over time. For example, sealing the first substance from the atmosphere may increase retention of the shape and consistency of the first substance over time. Without being bound by theory, enclosing the first substance within a smaller volume allows the system to quickly reach saturation (condensation and evaporation rates become equal) and therefore exposes the first substance to consistent moisture level, resulting in effectively zero net evaporation from the first substance. In some embodiments, the first substance is sealed from the atmosphere with a lid, such as a transparent lid, e.g. an acrylic lid. In particular embodiments, the first substance is enclosed within a plate, for example a transparent plate, and is sealed from the atmosphere with a lid, such as a transparent lid. In some embodiments, the plate and lid are acrylic. For the avoidance of doubt, where the first substance is in contact with at least one antimicrobial and/or at least one microbial (including a sample suspected of comprising microbials), the first substance and the at least one antimicrobial and/or at least one microbial may be sealed from the atmosphere.

In one embodiment, the device/system of the first aspect of the invention further comprises at least one antimicrobial in contact with the first substance. The device/system of the invention is suitable for assessing the antimicrobial susceptibility of microbes to any antimicrobial.

When the microbes are bacteria, the device/system of the first aspect is suitable for measuring the antibiotic susceptibility of bacteria to any antibiotic. Sometimes, the antibiotic is any one or a combination selected from the group consisting of amoxicillin, oxacillin, methicillin, ampicillin, chloramphenicol, gentamicin, streptomycin, carbenicillin, nitrofurantoin, trimethoprim, ciprofloxacin, temocillin, ofloxacin, cephalexin, co-amoxiclav or gentamicin. For example, the antibiotic may be any one or a combination selected from the group consisting of amoxicillin, oxacillin, methicillin, ampicillin, chloramphenicol, gentamicin, streptomycin and carbenicillin. Sometimes, the antibiotic comprises a $\beta$-lactam ring and/or an amido. The antibiotic may be any one or a combination selected from the group consisting of amoxicillin and oxacillin. Sometimes, the antibiotic is one type of antibiotic. However, combinations of antibiotics are sometimes more effective in the treatment of bacterial infection than individual antibiotics. The device/system may be used to measure the antibiotic susceptibility of bacteria to a combination of antibiotics, and is not restricted in the number of different antibiotics that may make up such a combination.

For the avoidance of doubt, "measuring" is used herein to refer qualitative or quantitative detection. For example, the device/system of the invention is suitable for qualitatively detecting antimicrobial susceptibility of microbes. The device/system may be used to assess whether or not microbes are susceptible to an antimicrobial. The device/system of the invention is also suitable for quantitatively detecting antimicrobial susceptibility of microbes. For example, the device/system may be used to assess the degree of susceptibility of microbes to an antimicrobial, such as the decrease in the rate of microbial growth or the rate of microbial cell death.

The device/system of the invention comprises an electrode system comprising two or more electrodes and a first substance suitable for electrical conductance and supporting microbial growth, wherein the first substance is in contact with the electrodes of the electrode system.

The electrode system may be any system suitable for the application of a potential difference across the first substance. To supply a potential difference, the electrode system comprises a minimum of two electrodes. A working electrode applies the desired potential to the first substance and transports charge to (thereby acting as an anode and reducing the analyte) or from (thereby acting as a cathode and oxidising the analyte) the first substance. A second electrode is required with a known potential, with which to use as a reference, and to complete the circuit and balance the charge, i.e. if the working electrode acts as an anode and reduces the analyte, then the second electrode balances the charge by oxidising a component of the first substance, thereby acting as a cathode. The second electrode acts as both a reference and a counter electrode.

Typically, the electrode system comprises 2 to 4 electrodes, often 3 or 4. Commonly, the electrode system comprises 3 electrodes. When the electrode system comprises 3 electrodes, it comprises a working electrode, a reference electrode (of a known potential and with which to use as a reference) and a counter electrode (to complete the circuit and balance the charge). In such an electrode system, changes in the potential difference at the working electrode are measured independently to the changes in the potential difference at the counter electrode. This set-up is advantageous over a 2 electrode system because analysis of the electrical response is simpler.

When the electrode system comprises 4 electrodes, it comprises a working electrode, a reference electrode, a counter electrode and a working sense electrode. In this set-up, the potential difference is measured at the working sense electrode (relative to the reference electrode), and is independent to the electrochemical reaction occurring at the working electrode, i.e. the effect of an applied current on the first substance itself is being measured. Such a set-up is useful for the measure of impedance across the first substance (discussed below).

The electrodes of the electrode system may be made of any material suitable for conducting electrons. It is preferable that the material is resistant to corrosion, and is able to conduct a suitable current load. Sometimes, the current load required is in the range of $\pm$ 1 nA to $\pm$ 1 mA; $\pm$ 10 nA to $\pm$ 0.1 mA; or $\pm$ 100 nA to $\pm$ 0.01 mA (with an error of $\pm$ 1%). Suitable materials include any one or a selection from the group consisting of gold, silver, platinum, palladium, titanium, graphite, carbon, brass, tungsten, ruthenium, iridium, titanium, nickel, aluminium, tin, or one or a selection of their oxides. Often, the electrodes of the system are made from any one or a selection from the group consisting of gold, silver, platinum, palladium, titanium, graphite and carbon. Typically, the electrodes are made from any one or a selection from the group consisting of gold, silver, platinum and palladium, preferably gold and silver. Commonly, the electrodes are made of one type of material, i.e. they are not made of a selection of materials.

The electrode system may be produced via additive printing processes, such as 3D-printing or screen-printing. These are suitable approaches for the production of cost-effective electrode systems and sensors (Tan, C., Nasir, M. Z. M., Ambrosi, A., Pumera, M., 2017. Anal. Chem. 89, 8995-9001). The electrode system may be microfabricated and may be produced by depositing the desired material, patterning the material with the desired micro features (e.g. by UV photolithography), and if necessary, removing or etching material. Preferably, the electrode system is screen-printed. Screen printed electrodes (SPEs) feature many advantages over more traditional electrodes such as ease of fabrication and cleaning procedures, reliability, low-cost, repeatability and provide rapid time to result. SPEs are amenable to mass production, whereby a large volume of electrodes can be produced at relatively low-cost compared to traditional macro or microelectrodes (Hayat, A., Marty, J. L., 2014. Sensors. 14, 10432-10453). Due to these advantages, SPEs lend themselves nicely to prototyping and for the development of novel sensing technologies, as reported here.

The device of the invention may be used for measuring antimicrobial susceptibility of microbes. Typically, the microbes are bacteria and the device is used for measuring antibiotic susceptibility of bacteria. The device is suitable for assessing the antibiotic susceptibility of any bacteria, i.e. any species and/or strain of bacteria. This is advantageous over approaches, e.g. genetic approaches, where specific DNA probes must be designed to detect a particular organism. Often, the identity of the species and/or strain of bacteria used is unknown. The species or strain of bacteria may belong to the Terrabacteria taxon. Sometimes, the species or strain of bacteria is gram-positive, i.e. the bacteria have a relatively thick peptidoglycan layer. The species or strain may belong to the Firmicutes phylum, and the Bacilli class. Sometimes, the species or strain belongs to the Bacillales order, sometimes the Staphylococcaceae family, and sometimes the Staphylococcus genus. Sometimes, the strain of bacteria belongs to the *Staphylococcus aureus* (*S. aureus*) species.

Conversely, the device of the invention may be used to screen microbes for those that are susceptible to a particular antimicrobial. For example, known species and/or strains of microbe with known drug resistance profiles may be contacted with the first substance of the device. The antimicrobial susceptibility of the known microbes to an antimicrobial may then be measured on contact of the first substance and known microbes with the antimicrobial.

*S. aureus* is a Gram-positive, coccoid bacterium found in the upper respiratory tract and on the skin (Taylor, T. A., Unakal, C. G., 2019. StatPearls, Treasure Island Publishing). *S. aureus* can cause skin infections, respiratory conditions and food poisoning (Tong, S. Y. C., David, J. S., Eichenberger, E., Holland, T. L., Fowler Jr, V. G., 2015. Clin. Microbiol. Rev. 28, 603-661). *S. aureus* is a useful bacterium to study the emergence of antibiotic-resistant strains, with Methicillin-resistant *S. aureus* (MRSA) being a common problem in the clinic (Harkins, C. P., Pichon, B., Doumith, M., Parkhill, J., Westh, H., Tomasz, A., de Lencastre, H., Bentley, S. D., Kearns, A. M., Holden, M. T. G., 2017. Genome. Biol. 18, 130). A useful biosensor should be able to distinguish between susceptible and resistant strains of *S. aureus* with the aim of providing the correct treatment to the patient as rapidly as possible.

Viewed from a third aspect, the invention provides a system comprising the device of the invention electronically connected to a potentiostat via the electrode system. The electronic connections may be any suitable connections to carry an electronic signal between the potentiostat and the electrode system. Typically, such electronic connections are electric wires. Each electrode of the electrode system is electronically connected to the potentiostat to allow the application and detection of electrical signals to and from each electrode.

The potentiostat of the system is suitable for at least EIS measurements. Preferably, the potentiostat of the system is suitable for at least EIS and DPV measurements. Sometimes, the potentiostat of the system is suitable for at least EIS, DPV, cyclic voltammetry and open circuit potentiometry measurements. As such, the potentiostat is capable of analysing electrical impedance as a function of test frequency, i.e. the potentiostat comprises an impedance analyser. Alternatively, an impedance analyser may be used separately to the potentiostat for EIS measurements. In some embodiments, the potentiostat of the system is suitable for square wave voltammetry (SWV) and chronoamperometry measurements. Accordingly, in some embodiments, the potentiostat of the system is suitable for at least EIS, DPV, cyclic voltammetry, open circuit potentiometry, SWV and chronoamperometry measurements.

EIS is capable of real-time data capture. The impedance of the electrode-first substance interface is studied using an alternating potential difference across a range of frequencies to establish information regarding the interface, its electron transfer properties and surrounding diffusional behaviour. The term "impedance" used herein, is a measure of the frequency dependant resistance of the first substance to a current flow of a circuit, and is calculated according to the formula below, where $E_\omega$ is equal to the frequency-dependant potential and $I_\omega$ is equal to the frequency-dependent current.

$$Z_\omega = \frac{E_\omega}{I_\omega}$$

Changes in impedance are reflective of changes in microbe growth as a function of time: as the metabolic rate and population of microbes increase, the frequency dependent resistance of the first substance to a current flow (i.e. the impedance) increases. Without being bound by theory, as the microbes grow and metabolise the constituents within the first substance, the balance of charged species in the first substance changes. Thus, measuring impedance over time gives an indication of the rate of microbe growth.

DPV can be used to sensitively investigate electron transfer to and from an electrode surface. An electric potential is measured between the working electrode and the reference electrode, while the current is measured between the working electrode and the counter electrode. The electric potential is increased or decreased linearly with time to a set potential (a potential linear sweep), or is incrementally increased or decreased with time (a staircase waveform). A staircase waveform is preferred. A series of regular voltage pulses are superimposed upon the potential linear sweep or staircase waveform. The current is measured before (initial current) and after (final current) the voltage pulse, and the difference between the final and initial current is plotted as a function of the applied electric potential. In this way, the effect of the non-Faradaic charging current is minimised, i.e. only the Faradaic current (the current generated by the reduction or oxidation of components of the first substance) is measured, thus electron transfer may be analysed more precisely.

The peak current ($I_{pk}$) measured in DPV plots corresponds to the current generated on oxidation of a component of the first solution. It is dependent on the resistance of the first solution. Therefore, changes in the peak current are reflective of changes in microbe growth as a function of time: as the population of microbes increases, the peak current decreases. Thus, measuring the peak current over time gives an indication of the rate of microbe growth.

The system of the invention may be used for measuring antimicrobial susceptibility of microbes. The skilled person will recognise that the embodiments and features described herein in relation to microbes of the invention also apply here. For example, the microbes may be bacteria, e.g. Gram-positive or Gram-negative bacteria, such as Staphylococcus aureus (*S. aureus*).

The device/system of the invention may be used to test a number of antimicrobial agents and in this manner a separate electrode system may be provided for each antimicrobial agent to be tested. In this manner the present invention may provide a library of devices of the invention (each device comprising a separate antimicrobial agent or combination of agents, as well as a device lacking an antimicrobial agent) and a potentiostat electronically connected to the electrodes of the electrode system of the device.

The skilled person will recognise that the embodiments and features related to the electronic connections of the third aspect of the invention also apply to other aspects and embodiments of the invention. For example, the electronic connections of any aspect may be any suitable to carry an electronic signal between the potentiostat and the electrode systems. The devices of the library may be used to assess the antimicrobial susceptibility of microbes to different antimicrobials.

Conveniently at least one device of the library may be used to measure a background electrical response, a first control electrical response and a second control electrical response (see below for further discussion of the background and control electrical responses).

The first aspect of the invention provides a method of measuring microbial growth comprising:

(i) contacting the device or system of the invention with at least one antimicrobial or candidate antimicrobial and a sample suspected of comprising or known to comprise microbes;
(ii) applying an electrical stimulus to the electrodes at least at a first and second time point; and
(iii) sensing an electrical response from the electrodes at the at least first and second time points;

wherein a difference in electrical response between the at least first and second time points is indicative of microbial growth.

The device or system of the invention is contacted with at least one antimicrobial and a sample suspected of comprising microbes.

The skilled person will recognise that the embodiments and features related to the first substance, antimicrobial, microbes and electrode system of the second or third aspects of the invention also apply to further aspects of the invention.

Antimicrobials and microbes may be contacted with the first substance by any suitable means. Sometimes, the at least one antimicrobial is contacted with the first substance prior to contacting with microbes. Sometimes, a solution comprising the at least one antimicrobial is contacted with the first substance. The solution may be aqueous. The solution comprising the at least one antimicrobial may be, for example pipetted, dropped, flowed or the like onto the first substance.

Following the contacting of the first substance with the at least one antimicrobial, a solution comprising microbes may be likewise contacted with the first substance. The solution may be aqueous and may comprise a microbial growth medium. When the microbes are bacteria, an aqueous solution comprising bacteria and LB medium may be pipetted directly onto the first substance.

The type of electrical signal applied to the electrode system depends on the measurement to be taken (e.g. EIS or DPV). As described above, EIS may be used to measure the impedance of the first solution and is studied using an alternating potential across a range of frequencies. Therefore, sometimes the electrical signal applied to the electrode system is an alternating potential. Sometimes the frequency of the alternating potential is in the range of about 150 Hz to about 0.05 Hz. Sometimes, about 120 Hz to about 0.07 Hz or about 100 Hz to 0.1 Hz. The amplitude of the waveform of the alternating potential is sometimes about 20 to about 5 mV rms, other times 15 to about 7 mV rms, or about 10 mV rms.

DPV may be used to measure electron transfer to or from a working electrode surface. As described above, an electric potential is increased linearly with time to a set potential (a potential linear sweep), or is incrementally increased with time (a staircase waveform). A series of regular voltage pulses are superimposed upon the potential linear sweep or staircase waveform. Therefore, sometimes the electrical signal applied to the electrode system is a series of regular voltage pulses superimposed upon the potential linear sweep or staircase waveform, with a potential applied in the range of about -1.0 V to 1.0 V. Other times, the potential applied is in the range of about -0.5 V to 0.7 V or about - 0.3 V to 0.5 V.

In one embodiment, the electrical stimulus comprises a potential applied between the working electrode and the reference electrode and the electrical response consists of a current between the working electrode and the counter electrode. The type of electrical response detected from the electrode system depends on whether the measurement is EIS or DPV. If the measurement is EIS, then the electrical response detected is an alternating current (AC). Thus, when measuring EIS, the electrical signal applied is an alternating potential and the electrical response is an AC. If the measurement is DPV, then the electrical response is a direct current (DC). The current is measured before (initial current) and after (final current) the voltage pulse. Thus, when measuring DPV, the electrical signal applied is a series of regular voltage pulses superimposed upon the potential linear sweep or staircase waveform and the electrical response is a direct current measured before and after the voltage pulse.

As defined herein, time points are equal to the times at which an electrical stimulus is applied to the electrodes and an electrical response is sensed from the electrodes. The first time point may be at any time from contact of the microbes and antimicrobial with the first substance. For example, the first time point may be 0 to 150 minutes after contact of the microbes and antimicrobial with the first substance. The at least second time point is collected after the first time point, for example the second time point may be collected days or hours after the first time point. In one embodiment, the second time point is collected 1 to 150 minutes after the first time point, for example 1 to 60 minutes, 1 to 40 minutes or 1 to 30 minutes.

The skilled person is aware that an electrical stimulus may be applied to the electrodes and an electrical response may be sensed from the electrodes at more than two time points. The skilled person is also aware that the collection of more time points leads to greater certainty of microbial growth. The more than two time points may be collected at any time interval between the first and at least second time point. The at least second time point is defined herein as the final time point.

Where the system of the invention comprises two or more devices, the two or more devices may be used to measure the antimicrobial susceptibility of the sample suspected of comprising microbes to two or more different antimicrobials. Alternatively, the two or more devices may be used to measure the antimicrobial susceptibility of the sample suspected of comprising microbes to two or more different doses of the same antimicrobial. The doses may range from 1 ng/ml to 100 mg/ml, for example 0.5 μg/ml to 0.5 mg/ml, 1 μg/ml to 100 μg/ml, 4 μg/ml to 70 μg/ml, 6 μg/ml to 60 μg/ml or 8 μg/ml to 50 μg/ml.

Where the system of the invention comprises four or more devices, two or more devices may be used to measure the antimicrobial susceptibility of the sample suspected of comprising microbes to two or more different antimicrobials and two or more devices may be used to measure the antimicrobial susceptibility of the sample suspected of comprising microbes to two or more different doses of the same antimicrobial.

Conversely, where the system of the invention comprises two or more devices, the two or more devices may be used to screen microbes for those that are susceptible to a particular antimicrobial. For example, at least two known species and/or strains of microbe may be contacted with the first substance of the two or more devices. The antimicrobial susceptibility of the different known microbes to an antimicrobial or candidate antimicrobial may then be measured on contact of the first substance and known microbes with the antimicrobial.

Differences between the electrical responses recorded from susceptible microbials and resistant microbials may manifest at the same time point. There may be a consistent pattern of differences between the responses of the microbials across the frequency profile collected at the same time point. For example, when the electrical stimulus comprises an alternating potential, one particular frequency of electrical stimulus may generate electrical responses from susceptible or resistant microbials that differ from each other.

In one embodiment, the method of the invention additionally comprises measuring a background electrical response. This comprises:

(i) contacting the device or system of the invention with at least one antimicrobial;
(ii) applying an electrical stimulus to the electrodes at least at a first and second time point; and
(iii) sensing a background electrical response from the electrodes at the at least first and second time points;

wherein a difference in background electrical response between the at least first and second time points is background signal.

The background electrical response is the electrical response of the system in the absence of microbes. "Absence" refers to the case where no microbes have been intentionally contacted with the first substance. Therefore, a background electrical response should be measured before the first substance of the system is contacted with microbes. Alternatively, where a system of the invention comprises two or more devices of the invention, one such device may be used to measure microbial growth and the other may be used to measure a background signal in response to the addition of a dummy sample or a clinical sample known to be free of microbes.

The background electrical response may be subtracted from the electrical response. If the time periods used to measure the background electrical response are identical to those used to measure the electrical response then the background electrical response may be subtracted from the electrical response for each time period measured. Subtracting the background response from the electrical response allows any changes in electrical response caused by microbial growth to stand out from the background electrical response of the system.

In another embodiment, the method of the invention additionally comprises measuring a first control electrical response. This comprises:

(i) contacting the device or system of the invention with a sample suspected of comprising microbes;
(ii) applying an electrical stimulus to the electrodes at least at a first and second time point; and
(iii) sensing a first control electrical response from the electrodes at the at least first and second time points;

wherein a difference in first control electrical response between the at least first and second time points is first control signal.

The first control electrical response is the electrical response of the system in the presence of microbes and in the absence of antimicrobials, i.e. where no antimicrobials have been intentionally contacted with the first substance. The purpose of the first control electrical response is quality control, to check that microbial growth is possible on the system set up (i.e. that the first substance is set up correctly for microbial growth) and to ensure that microbial growth leads to a detectable electrical response (i.e. that the electrode system and potentiostat are set up correctly). A positive first control electrical response gives an indication that devices of the same batch are in working order.

Where the system of the invention comprises two or more devices of the invention, one such device may be used to measure microbial growth and another may be used to measure a first control signal. In addition to quality control, the first control signal may be used to assess which data give a greater certainty of microbial growth. If microbes are present in the sample suspected of comprising microbes, their concentration is likely to be unknown. Typically, infected wounds comprise around 500,000 microbes/mL. In theory, a higher concentration of microbes leads to a greater change in electrical signal resulting from microbial growth (more microbes metabolise the components of the first substance, leading to a greater change in the balance of charged species), which in turn leads to greater certainty of growth from the data collected. Where the concentration of microbes is low, the change in electrical signal resulting from microbial growth may be weak, leading to uncertainty in the data collected.

The first control signal may be used to give an indication of the certainty time point, at which the change in electrical signal is enough to be certain of microbial growth, for example the time point at which the change in electrical signal is greater than 3 times the standard deviation of the background signal. Data collected from the device used to measure microbial growth at time points greater than the certainty time point provide an accurate measure of antimicrobial susceptibility of the microbes.

The skilled person is aware that the device/system of the invention may be used to measure the antimicrobial susceptibility of a sample suspected of comprising microbes where the microbes in the sample suspected of comprising microbes are at any concentration. A lower microbe concentration may lead to data collection over a longer period of time, i.e. the at least second time point may be greater than 150 minutes after the first time point, whereas a higher microbe concentration may lead to a quicker measure of antimicrobial susceptibility.

In one embodiment, the method of the invention comprises using at least one device to measure microbial growth, using at least one device to measure a first control electrical response and comparing the electrical responses of the at least two devices.

Where the system of the invention comprises three or more devices of the invention, one such device may be used to measure microbial growth, a second device may be used to measure a first control signal and a third device may be used to measure a background signal.

Thus, in a further embodiment, the method of the invention comprises using at least one device to measure microbial growth, using at least one device to measure a first control electrical response, using at least one device to measure a background signal, and comparing the electrical responses of the at least three devices.

Sometimes, the first control electrical response is measured and subtracted from the electrical response. This allows any changes in electrical response caused by antimicrobial susceptibility to stand out from the first control electrical response of the system. The skilled person is aware that other analytical techniques in addition or instead of the subtraction technique may be used to analyse the electrical responses. Other analytical techniques include principal component analysis and machine learning algorithms.

In a further embodiment, the method of the first aspect additionally comprises measuring a second control electrical response, which comprises:

(i) contacting the device or system of the invention with at least one antimicrobial and microbes known to be susceptible to the at least one antimicrobial;
(ii) applying an electrical stimulus to the electrodes at least at a first and second time point; and
(iii) sensing a second control electrical response from the electrodes at the at least first and second time points;

wherein a difference in second control electrical response between the at least first and second time points is second control signal.

The second control electrical response is the electrical response of the system in the presence of at least one antimicrobial and microbes known to be susceptible to the at least one antimicrobial, i.e. where one would expect microbial growth to be inhibited. The second control electrical response is for quality control purposes and checks that the inhibition of microbial growth is detectable on the system set up. A positive second control electrical response gives an indication that devices of the same batch are in working order.

Where the system of the invention comprises four or more devices of the invention, one such device may be used to measure microbial growth, a second device may be used to measure a first control signal, a third device may be used to measure a background signal, and a fourth device may be used to measure a second control signal.

In one embodiment, the method of the invention comprises using at least one device to measure microbial growth, a first control signal, a background signal, and/or a second control signal.

In one embodiment, the method of the invention comprises using at least one device to measure microbial growth, using at least one device to measure a first control signal, using at least one device to measure a background signal, using at least one device to measure a second control signal and comparing the electrical responses of the at least four devices.

Although the majority of the specification is directed to a device or system that is useful for detecting microbial growth in order to ascertain the antimicrobial susceptibility of microbes, the skilled person is aware that the same device or system of the invention may be used to detect microbial growth in order to ascertain the antimicrobial properties of substances. For example, extracts with unknown antimicrobial properties, or candidate antimicrobial agents may be tested for their ability to inhibit the growth of one or more microbes. Said one or more microbes may in embodiments be one or more Gram positive bacteria and/or one or more Gram negative bacteria.

Thus, in a specific aspect, the invention provides a method of measuring microbial growth comprising:

(i) contacting the device or system of the invention with at least one microbe and a sample suspected of comprising antimicrobials;
(ii) applying an electrical stimulus to the electrodes at least at a first and second time point; and
(iii) sensing an electrical response from the electrodes at the at least first and second time points;

wherein a difference in electrical response between the at least first and second time points is indicative of microbial growth.

The skilled person is aware that, as in the first aspect, this method may additionally comprise measuring a background electrical response, a first control electrical response and/or a second control electrical response. In the case of measuring a background response, the at least one antimicrobial is replaced with the sample suspected of comprising antimicrobials; and in the case of measuring a first control electrical response, the sample suspected of comprising microbes is replaced with the at least one microbe.

Viewed from a fourth aspect, the invention provides a kit-of-parts comprising:

(i) at least one device or system of the invention; and
(ii) at least one antimicrobial.

The kit-of-parts may comprise more than one device or system of the invention and more than one type of antimicrobial selected from the group consisting of β-lactam antibiotics, macrolides, lincosamides, aminoglycosides, tetracyclines, vancomycin, sulphonamides, fluoroquinolones, chloramphenicol, nitrofurantoin and isoniazid.

In accordance with the fourth aspect of the invention, the first substance of the device or system is not in contact with the at least one antimicrobial and may be contacted by the user of the kit. The skilled person is aware that one or more devices or systems of the invention may be provided to the user with one or more antimicrobials pre-loaded onto the first substance, i.e. the one or more antimicrobials may be in contact with the first substances of the one or more devices or systems. For example, the kit may comprise an array of devices, each device pre-loaded with one or more antimicrobials specific to a certain microbial. The array of devices may be organised into infection types such that devices comprising one or more antimicrobials known to be effective against microbes of a certain infection type are grouped together. For example, devices comprising one or more antimicrobials known to be effective against urinary tract infections may be grouped together, whilst those comprising antimicrobials known to be effective against skin infections may be grouped together, separate from the other devices.

The embodiments and features related to the device, antimicrobial, potentiostat, electrical contacts and microbes of the first to third aspects of the invention also apply to the fourth aspect of the invention.

The first substance may be formed by contacting an electrolyte, gel suitable for supporting microbe growth and a redox mediator. These components may be contacted in any order.

The redox mediator, electrolyte and gel suitable for supporting microbe growth may be contacted at room temperature to form an initial substance. Sometimes, the initial substance is heated to temperatures of about 50 to about 150 °C prior to addition of the at least one antimicrobial. The initial substance may be heated to temperatures of about 70 to about 140 °C, sometimes about 110 to about 130 °C, prior to addition of the at least one antimicrobial.

After heating, the initial substance may be cooled prior to addition of the at least one antimicrobial. This avoids inactivation of the at least one antimicrobial. Sometimes, the initial substance is cooled to temperatures of about 20 to about 50 °C, such as 20 to 40 °C. The first substance may be re-melted prior to contacting with the electrode system and forming the device. To prevent any changes in the consistency of the initial substance, the device may be stored in a controlled environment, for example a sealed container, before use. The at least one antimicrobial may be contacted with the first substance of the device and stored prior to contacting the first substance with microbes. To prevent any changes in the consistency of the first substance and/or antimicrobial degradation, the device, once contacted with the at least one antimicrobial, may be stored in a controlled environment, for example in a sealed container.

Prior to the addition of microbes, the first substance may be stabilized at 37 °C for 5 to 30 minutes, sometimes 5 to 20 minutes, such as 10 minutes. The device may be sealed during use, in a controlled environment at 37 °C within a humidity chamber and within an incubator.

Any discussion herein of documents, acts, materials, devices, articles or the like is not to be taken as an admission that any or all of these matters form part of the prior art base or were common general knowledge in the field relevant to the present disclosure as it existed before the priority date of each claim of this application.

The person skilled in the art is to understand that the present embodiments may be read alone, or in combination, and may be combined with any one or a combination of the features described herein.

## EXAMPLES

The approach to measuring antibiotic susceptibility exemplified herein involves modifying a commercially available gold SPE (DropSens Au 223BT) via drop coating using an agarose-based hydrogel to produce a sensitive, mass manufacturable sensor system capable of determining the effectiveness of antibiotics for *S. aureus* and MRSA.

Since MRSA is a leading cause of surgical, hospital and community acquired infection (Coll, F., Harrison, et al., 2017. Sci. Transl. Med. 9, 1-19), distinguishing between antibiotic-sensitive *S. aureus* vs MRSA has been investigated herein. To assess antibiotic susceptibility, electrochemically derived growth profiles for antibiotic-sensitive *S. aureus* and MRSA have been measured using electrodes modified with agarose-gel deposits containing growth medium and different concentrations of antibiotics. The optimum electrochemical parameters were evaluated with regards to sensitivity of antibiotic-sensitive *S. aureus* and MRSA growth, and reported as a function of bacterial growth for both strains tested and in the presence and absence of two commonly used antibiotics: amoxicillin and oxacillin.

## 1. MATERIALS AND METHODS

Commercially available Gold (Au) screen printed electrodes (SPEs) with on-chip silver reference and gold counter electrodes were obtained from DropSens (Oviedo, Spain) (ref 223BT).

Gels for electrode characterisation contained agarose, Miller LB Broth, 200 mM KCL + 1 mM Fe[CN]$_6{}^{3-}$ + 1 mM Fe[CN]$_6{}^{4-}$ (Ferri-Ferro Cyanide (FF-C) solution) with some containing antibiotics (amoxicillin or oxacillin at 8$\mu$g/ml in deionised (DI) water). All of these chemicals were purchased from Sigma Aldrich, Dorset, UK. Gel components were initially mixed at room temperature, and then autoclaved at 121°C to both sterilise and allow the agarose to mix. Upon cooling, the gels hardened, and were re-melted prior to deposition on the electrode. Antibiotics were added to the gels upon cooling to avoid inactivation at high temperature.

Bacterial plates for culture contained LB media + agar (Sigma Aldrich). Bacterial strains *Staphylococcus aureus* (ATCC 29213) and methicillin-resistant *Staphylococcus aureus* (MRSA: ATCC 43300) were streaked out onto LB agar-plates from a freshly prepared frozen glycerol stock of each (stored at -80°C). Single colonies were used to inoculate overnight cultures of Lysogeny Broth (5 ml at 37°C). Bacteria from the overnight cultures were directly pipetted onto the gels on the electrode surface, at a volume of 5 $\mu$l and a bacterial concentration ~ 10$^7$ CFU/ml (CFU - colony forming unit) giving a final count on the sensor of ~50,000 CFUs. LB agarose was prepared as LB but replacing agar with agarose.

Prior to measurement, all electrodes were cleaned using an electrochemical method involving immersion of the electrode in DI water + 200 mM KCL + 1 mM Fe[CN]$_6{}^{3-}$ + 1 mM Fe[CN]$_6{}^{4-}$ (Ferri-Ferro Cyanide (FF-C) solution) and performing cyclic voltammetry (CV) over a potential range of -0.3 V to + 0.8 V for 10 scans at 100 mV/s. After cleaning, the electrodes were rinsed with DI water and dried using an aerosol gun prior to use.

### 1.2 Characterisation

All electrode measurements were performed using a three-electrode cell. DropSens SPEs are designed with the counter, reference and working electrodes together on a single chip, which eliminates the need to incorporate external counter and reference electrodes. All measurements were carried out using a potentiostat (PalmSens PS4, PalmSens, Houten, Netherlands).

CV measurements were performed by sweeping the potential between -0.3 V to 0.5 V with reference to the Ag electrode three times. Analysis was performed using the third measurement. Electrodes were also characterised using differential pulse voltammetry (DPV) across the same potential range as CV. Peak current ($I_{pk}$) was extracted from the DPV measurement. Electrodes were also characterised using EIS. The EIS response was measured across a frequency range between 100 kHz and 0.1 Hz at open circuit with a wavelength amplitude of 10 mV rms. The Nyquist and Bode impedance plots that were generated were fitted to the Randles' equivalent circuit to extract various impedance parameters. Figure 1 (b) shows an example EIS response, with the Randles' equivalent circuit used for data fitting shown in the inset.

Prior to bacterial deposition on the electrode surface, CV, DPV and EIS measurements were performed on the modified electrodes. Once deposited, the gel was left to partially stabilise at 37 °C for 10 minutes. Then, 5 $\mu$l of either *S. aureus* or MRSA culture from an overnight culture (~ 10$^7$ CFU/ml) was pipetted on top of the gel. DPV and EIS measurements were performed every 5 minutes using a measurement script, and extracted parameters were plotted as a function of time up to a maximum of 2.5 hours of bacterial growth post deposition. All bacterial growth experiments were performed at 37°C within a humidity chamber contained within an incubator (Genlab Ltd, Widnes, UK). Figure 1 (a (i)) displays the measurement setup adopted, including the Au electrode modified with a gel deposit connected to the potentiostat and related software for electrochemical measurements.

Minimum inhibitory concentrations (MIC) of antibiotics were determined by two-fold broth microdilution in 96-well microliter plates. Aliquots (100 $\mu$l) of cation-adjusted Mueller Hinton broth (Barry, A. L. et al., 1992. J. Clin. Microbiol. 30, 585-589); Oxoid CM0405), containing serial dilutions of antibiotic, were inoculated with 5 $\times$ 10$^4$ CFU/well of bacteria and incubated at 37 °C for 17 h. Growth in each well was determined by measuring the optical density of the well at a wavelength of 600 nm (OD$_{600}$) using a SpectraMax 190 plate reader (Molecular Devices) with SoftMax Pro 7.1 software (Molecular Devices). Percentage of growth inhibition for each well was calculated from negative controls (media only) and positive controls (bacteria without antibiotic). MIC80 was defined as the highest dilution of antibiotic which resulted in > 80% of inhibition of growth (n = 4, Z > 2.5).

## 2. RESULTS AND DISCUSSION

### 2.1 Electrodes and Electrochemical Measurements

A commercially available electrode was chosen for this study in order to benefit from its low-cost and ease of integration with the developed measurement setup. The electrode chosen was a 1.6 mm-diameter screen printed electrode (SPE) (DropSens, Oviedo, Spain). The SPE (33 mm × 10 mm × 0.5 mm (length × width × height)) consists of three main parts: a 1.6 mm-diameter Au working electrode, Au counter electrode and a silver (Ag) reference electrode. The electrical contacts on the electrode are made of Ag. Previously, these Au SPEs have been used to produce disposable nucleic acid biosensors (Kuralay, F. et al., J., 2015. Talanta. 85, 1330-1337) and have been used for the detection of volatile fatty acids (Ndiaye, A. L. et al., 2016. Biosensors. (Basel). 6, 46), amongst other applications.

Figure 1 (b) shows a typical EIS measurement performed from 100 kHz to 0.1 Hz, with the inset displaying the classical Randles' equivalent circuit model used to fit the EIS data. By fitting the data to this circuit, a number of analytical parameters can be extracted, including the solution resistance ($R_{SOLN}$), charge transfer resistance ($R_{CT}$) and double layer capacitance ($C_{DL}$, the capacitance at the interface between the working electrode and the first substance). In addition, through extraction of the EIS Bode plot, information regarding the impedance ($Z$) at different frequencies and the phase angle can be extracted. A wide range of parameters were found to change over time as a function of microbial growth, and subsequent results present data extracted using the most sensitive and convenient parameters. In addition, the DPV peak current ($I_{pk}$) can also be investigated as a function of bacterial growth.

## 2.2 Gel Production and Electrode Preparation

Before measuring bacterial growth on the electrodes, the composition and volume of the gel forming solution was investigated in order to establish the optimum sensor modification protocol for functionalisation and electrochemical measurement. Agarose was chosen as a suitable material, due to its UV transparency and low toxicity. Even small changes in gel concentration can affect its properties such as gelation and other properties of the 3D construct can be significantly affected, therefore, three similar concentrations of agarose were tested (1 wt%, 1.5 wt% and 3 wt%) to evaluate gel stability and ease of deposition, with the aim to select the optimum agarose concentration for the application. Figure 1 (a (ii)) shows an example gel deposit on the electrode. Figure 1 (c) shows the electrochemical response (i) CV and (ii) EIS of the three agarose gels tested. Each gel contained FF-C to ensure a Faradaic signal could be measured, i.e. the FF-C redox mediator could permeate the gel effectively to reach the working electrode surface. The redox agent was added to the gel in order to expand the number of electrochemical parameters that could be investigated during microbial growth. The CV response (Figure 1 (c) (i)), shows that as the concentration of agarose in the gel is increased, the response current decreased, with greater separation between the oxidation and reduction peaks, indicating an improved electrochemical response for the 1 wt% gel. This is confirmed with EIS measurements (Figure 1 (c) (ii), whereby $R_{CT}$ increases significantly as agarose concentration is increased.

The Randles-Sevcik equation can be used to describe the effect of scan rate on peak current for cyclic voltammetry measurements:

$$i_p = 0.4463nFAC \left(\frac{nFvD}{RT}\right)^{1/2},$$

where $i_p$ is the maximum current in amps, $n$ is the number of electrons transferred in the redox event, $A$ is the electrode area in cm$^2$, $F$ is the Faraday Constant in C mol$^{-1}$, $D$ is the diffusion coefficient in cm$^2$ s$^{-1}$, $C$ is the concentration in mol cm$^{-3}$, $v$ is the scan rate in V.s$^{-1}$, $R$ is the gas constant in J K$^{-1}$ mol$^{-1}$ and $T$ is the temperature in K.

Various parameters can be extracted from CV curves including $i_p$ and peak potential separation ($\Delta E_p$). Table 1 displays these parameters for each agarose gel concentration, and it is clear that as the concentration of agarose in the gel is increased, there is a significant increase in the peak separation, indicative of reduced reversibility at higher agarose concentrations.

From the EIS data, the equation below can be used to find the diffusion coefficient of the ionic species (FF-C) or the area of the electrode:

$$R_{CT} = \frac{4RTl}{ADF^2C},$$

where $l$ is the diffusion length and $R_{CT}$ is the charge transfer resistance in ohms extracted from a fit of the measured EIS data. By rearranging this equation for $D$, the diffusion coefficient at each agarose concentration can be determined, and used to assess the influence agarose has on the effect of FF-C to reach the electrode surface under mass transport conditions. '$D$' was chosen as the parameter of interest in this case and the electrode area was kept constant due to the cross-linked nature of the gel, meaning '$D$' would likely have a greater impact on the diffusional behaviour rather than the resulting electrode area. However, it is acknowledged that equally '$D$' could have been fixed and the area as a function of agarose concentration could have been investigated instead.

Table 1 displays the value of $D$ extracted at each agarose concentration, ranging from $4.15 \times 10^{-9}$ for 1 wt%

agarose to $7.64 \times 10^{-10}$ cm$^2$ s$^{-1}$ for 3% agarose. The 1.5 wt% agarose displays a significantly higher value of $2.23 \times 10^{-6}$ cm$^2$ s$^{-1}$, however, it also displays a greater $R_{SOLN}$ from the EIS measurement which helps explains the difference seen in the calculated value of $D$. From Table 1 it can also be noted that the $\chi^2$ value, or the 'goodness of fit', statistical test is around an order of magnitude higher for the 1.5 wt% agarose, compared to the other two agarose concentrations tested. This is an indication that while the 1.5 wt% gel measurement may not be entirely consistent with the others, it is still useful as means to show the gel development. Due to the improvement in electrochemical response seen at the lower agarose concentration and its ease at forming a reproducible spherical gel on the electrode surface, the 1 wt% gel was chosen for all subsequent experiments.

The CV and EIS response of three gels deposited using dip-coating and drop-casting (10 and 20 $\mu$l) are shown in Figure 1 (d) (i) and (ii) respectively. Two drop-casting volumes were tested; pipetting 10 $\mu$l onto the WE only, and then 20 $\mu$l pipetted onto all three electrodes (WE/RE/CE). From the CV response and the EIS, the 20 $\mu$l-gel deposit appears to produce the most consistent electrochemical response. In terms of the CV curves, the 20 $\mu$l-gel deposit produces around a 10 times greater peak current, and the peak separation is closer to the theoretical limit ($\sim$ 59 mV) at 85.55 mV, compared to 140.94 mV for the 10 $\mu$l-gel deposit. It also provides an acceptably low $R_{CT}$ enabling satisfactory electron transfer between the gel deposit and the electrode surface. Therefore, for subsequent measurements, at least 20 $\mu$l of gel was pipetted onto the SPE, to ensure coverage of all three electrodes on the chip. The larger gel volume also enables longer measurement times, since smaller volumes have a tendency to evaporate more readily during microbial growth.

Table 1 provides a summary of the electrochemical measurement data extracted from the gel development stage. The majority of the parameters are taken from EIS data, fitted to the Randles' equivalent circuit. The data confirms the results shown in Figure 1, that 20 $\mu$l, 1 wt%-agarose gels drop-casted onto the SPE provided the optimum electrochemical response, suitable for subsequent bacterial growth measurements.

**Table 1**. Summary of data shown in Figure 1. Table shows CV, EIS and DPV measurement data extracted from gel development experiments. Measurement parameters include $R_{SOLN}$, $R_{CT}$, $C_{DL}$, Z (100 kHz), Phase, DPV $I_{pk}$, CV $i_{pk}$, CV $\Delta E_p$ and D.

| Experiment: Agarose Concentration (%) | $R_{SOLN}$ $(\Omega)$ | $R_{CT}$ $(k\Omega)$ | $C_{DL}$ $(\mu F)$ | $\chi^2$ | Z at 100 kHz $(\Omega)$ | Phase (°) | DPV $I_{pk}$ $(\mu A)$ | CV $i_{pk}$ $(\mu A)$ | CV $\Delta E_p$ (mV) | $D$ (cm$^2$ s$^{-1}$) |
|---|---|---|---|---|---|---|---|---|---|---|
| 1 % | 299.80 | 24.25 | 1.84 | 0.0007 | 415.4 0 | 22.42 | 0.46 | 0.69 | 110.40 | 4.15×10$^{-9}$ |
| 1.5 % | 731.40 | 94.03 | 0.57 | 0.0101 | 658.5 2 | 6.58 | 0.19 | 0.82 | 281.95 | 2.23×10$^{-6}$ |
| 3.0 % | 249.60 | 338.8 | 0.24 | 0.0011 | 246.6 5 | 6.18 | 0.02 | 0.41 | 498.52 | 7.64×10$^{-1}$ |
| **Experiment: Gel Deposition Method** | $R_{SOLN}$ $(\Omega)$ | $R_{CT}$ $(k\Omega)$ | $C_{DL}$ $(\mu F)$ | $\chi^2$ | Z at 100 kHz $(\Omega)$ | Phase (°) | DPV $I_{pk}$ $(\mu A)$ | CV $i_{pk}$ $(\mu A)$ | CV $\Delta E_p$ (mV) | $D$ (cm$^2$ s$^{-1}$) |
| Dip Coat | 226.50 | / | 2.04 | 0.0024 | 217.4 2 | 1.66 | 5.26 | / | / | / |
| Drop Cast (10 µl) | 191.40 | 0.08 | 3.38 | 0.0008 | 185.6 4 | 1.87 | 2.91 | 0.102 | 140.94 | / |
| Drop Cast (20 µl) | 242.2 | 3.91 | 1.36 | 0.0009 | 242.4 1 | 4.37 | 1.42 | 1.26 | 85.55 | / |

To be able to assess bacterial growth on the electrodes for the purposes of investigating antibiotic susceptibility, different antibiotics were incorporated into the agarose-gels, and bacterial growth in the presence and absence of antibiotics was monitored, to investigate the response of either sensitive *S. aureus* or MRSA to the antibiotic of interest. The antibiotics amoxicillin and oxacillin were principally used to investigate *S. aureus* or MRSA growth on the gel modified electrodes. They were also used to perform benchmark minimum inhibitory concentration (MIC) assays on both the sensitive and resistant *S. aureus* strains to determine the optimum antibiotic concentrations for susceptibility testing. In addition to the antibiotics, the gels also included the same nutrients included in LB agar, to promote bacterial growth. To ensure bacterial growth was possible on gels containing different antibiotics and LB Broth, CV measurements were performed prior to bacterial deposition to investigate the influence of the different gel components.

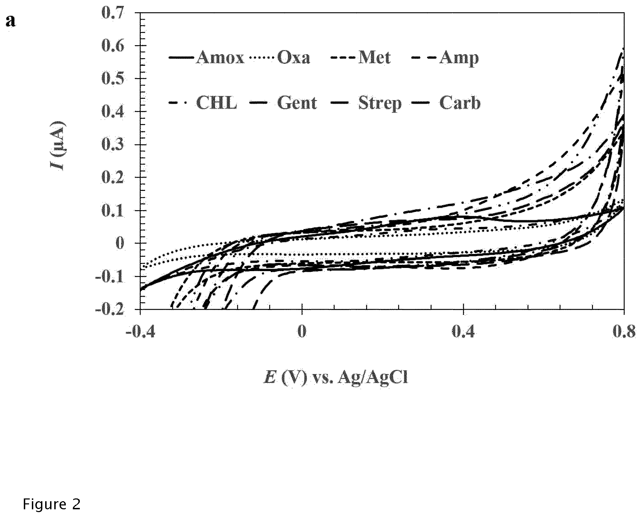

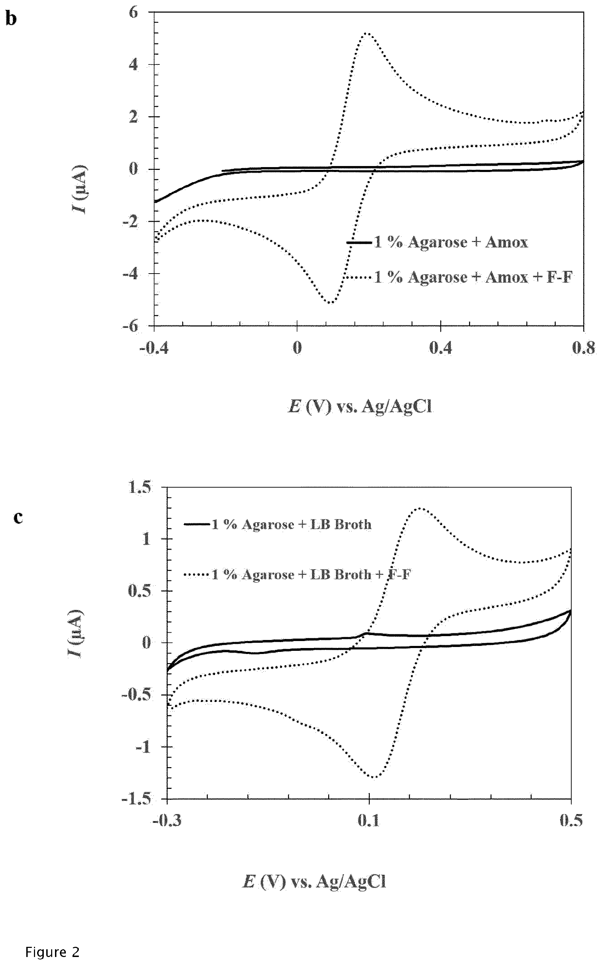

Figure 2 (a) shows the effect of various antibiotics (without gels) on the CV response of the SPE. A wide spectrum of antibiotics were tested including amoxicillin, oxacillin, methicillin, ampicillin, chloramphenicol, gentamicin, streptomycin and carbenicillin. In all cases, CV scans of the antibiotics produced no observable oxidation/reduction peaks which could potentially interfere with the FF-C electrochemical response when incorporated together into gels. Amoxicillin was then tested in an agarose gel with and without FF-C present (Figure 2 (b)). As expected, the CV response of the gel containing amoxicillin produced no electrochemical peaks, whereas the gel with both amoxicillin and FF-C showed the expected reversible appearing CV response, further confirming amoxicillin had no influence on the electrochemistry of the gel modified-SPE. Figure 2 (c) shows a similar experiment performed using the LB agarose gel with and without FF-C. Similarly, it is clear that FF-C is required to produce electrochemical signal, indicating LB itself had no influence on the response of the gel-modified electrode.

Table 2 provides a summary of the electrochemical measurement data extracted from the antibiotic screening experiment and electrochemical experiments performed on gels containing amoxicillin and LB. The majority of the parameters are taken from fitted EIS data alongside $I_{pk}$ from DPV measurements.

Table 2. Summary of data shown in Figure 2a-c. Table shows EIS and DPV measurement data extracted from antibiotic screening experiments on a range of commonly used antibiotics to evaluate potential electrochemical response and data from the gel composition experiments Measurement parameters include $R_{SOLN}$, $R_{CT}$, $C_{DL}$, Z (100 kHz), Phase and DPV $I_{pk}$.

| Antibiotic | $R_{SOLN}$ (k$\Omega$) | $R_{CT}$ (k$\Omega$) | $C_{DL}$ ($\mu$F) | Z at 100 kHz (k$\Omega$) | Phase (°) | PPV $I_{pk}$ ($\mu$A) |
|---|---|---|---|---|---|---|
| amoxicillin (AMOX) | 143.30 | / | 0.53 | 21.34 | 82.74 | 0.02 |
| oxacillin (OXA) | 49.81 | / | 0.28 | 22.79 | 111.17 | 0 |
| methicillin (MET) | 67.30 | / | 0.53 | 15.92 | 61.43 | 0.06 |
| ampicillin (AMP) | 40.93 | / | 0.38 | 10.88 | 67.63 | 0 |
| chloramphenicol (CHL) | 94.39 | / | 0.41 | 19.06 | 76.20 | 0.31 |
| gentamicin (GENT) | 7.97 | / | 0.77 | 6.05 | 31.68 | 0.01 |
| streptomycin (STREP) | 55.85 | / | 0.58 | 16.45 | 58.51 | 0 |
| catbenicillum (CARB) | 11.83 | / | 0.44 | 7.54 | 46.56 | 0 |
| **Gel Composition: Effect of Amoxicillin** | $R_{SOLN}$ ($\Omega$) | $R_{CT}$ (k$\Omega$) | $C_{DL}$ ($\mu$F) | Z at 100 kHz ($\Omega$) | Phase (°) | DPV $I_{pk}$ ($\mu$A) |
| Agarose + amox | 147.0 | / | 0.99 | 143.75 | 4.81 | 0.006 |
| Agarose + amox + F-F | 189.0 | 1.71 | 1.20 | 184.91 | 3.36 | 2.76 |
| **Gel Composition: Effect of LB Broth** | $R_{SOLN}$ ($\Omega$) | $R_{CT}$ (k$\Omega$) | $C_{DL}$ ($\mu$F) | Z at 100 kHz ($\Omega$) | Phase (°) | DPV $I_{pk}$ ($\mu$A) |
| Agarose + LB | 135.20 | / | 1.76 | 123.36 | 5.45 | 0.002 |
| Agarose + LB + F-F | 136.50 | 7.15 | 3.81 | 137.49 | 5.22 | 1.05 |

Initial testing of the electrodes within the incubator revealed 20 $\mu$l-volume gels had a tendency to completely evaporate within ~60 minutes, before meaningful growth data could be measured. Therefore, the gel volume was increased to 50 $\mu$l, which provided useful data for at least 120 - 150 minutes before complete evaporation. As shown in Table 3, this increase in volume had no influence on the gel itself or the electrochemistry of the SPE.

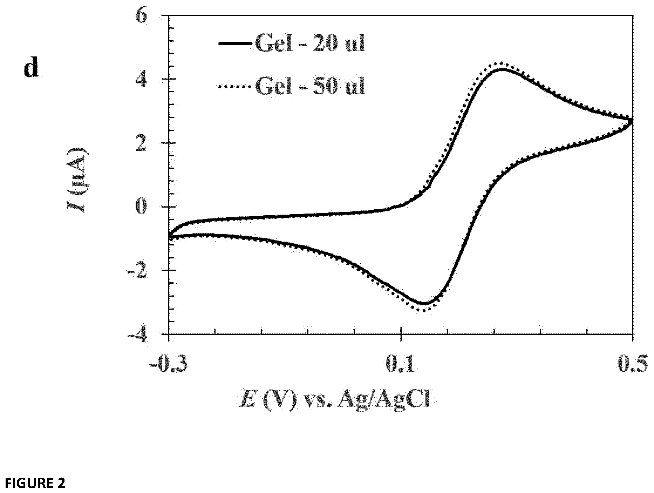

**Table 3.** Summary of data shown in Figure 2d. Table shows EIS and DPV measurement data extracted from gel volume experiments. Measurement parameters include $R_{SOLN}$, $R_{CT}$, $C_{DL}$, $Z$ (100 kHz), Phase and DPV $I_{pk}$.

| Experiment: Gel Volume ($\mu$l) | $R_{SOLN}$ ($\Omega$) | $R_{CT}$ (k$\Omega$) | $C_{DL}$ ($\mu$F) | $Z$ at 100 kHz ($\Omega$) | Phase (°) | DPV $I_{pk}$ ($\mu$A) |
|---|---|---|---|---|---|---|
| 20 | 58.56 | 4.99 | 1.39 | 53.36 | 7.22 | 2.57 |
| 50 | 59.89 | 5.25 | 9.17 | 65.64 | 7.89 | 1.48 |

## 2.3 Bacterial Growth Profiles

Baseline measurements were recorded, i.e. without any bacteria present, to characterise the electrochemical signal from the gel modified sensor as a function of time (up to 150 minutes). EIS/DPV measurements were taken every 5 minutes following gel deposition, and three curves were produced; a baseline, bacterial growth without antibiotic present, and bacterial growth in the presence of an antibiotic (amox or oxa). Antibiotics amox and oxa were prepared at a concentration of 8 $\mu$g/ml after evaluating their MICs to ensure MRSA could in fact grow on the electrode, but crucially that *S. aureus* growth would be inhibited. Differences between antibiotic-sensitive *S. aureus* and MRSA growth on antibiotic-free and antibiotic-containing gels were sought in order to assess susceptibility. From all the various parameters extracted from EIS and DPV measurements, it was found that the impedance (Z) at 100 kHz provided the most sensitive response to bacteria.

## 2.4 Amoxicillin modified sensor behaviour

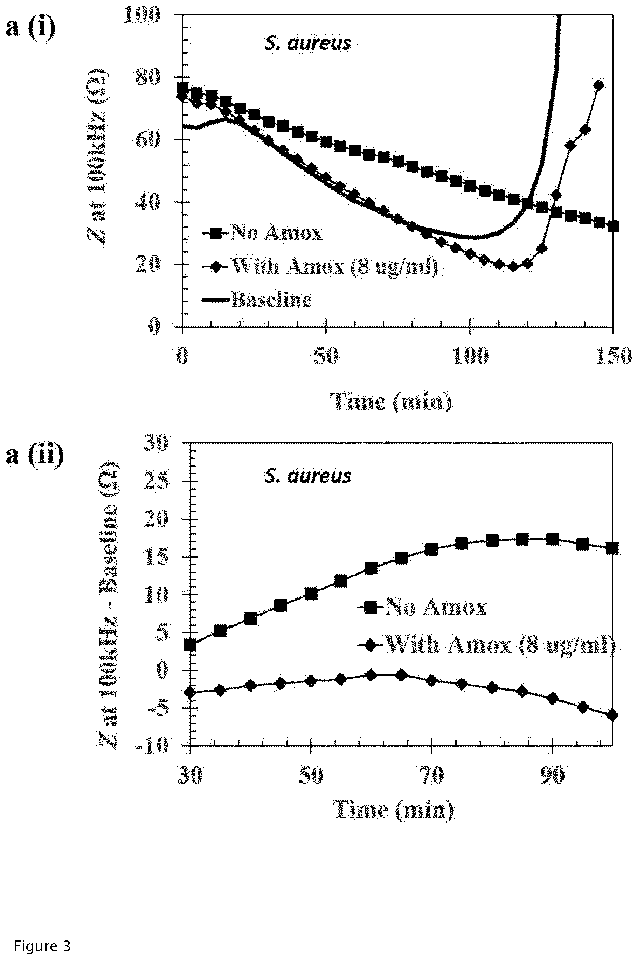

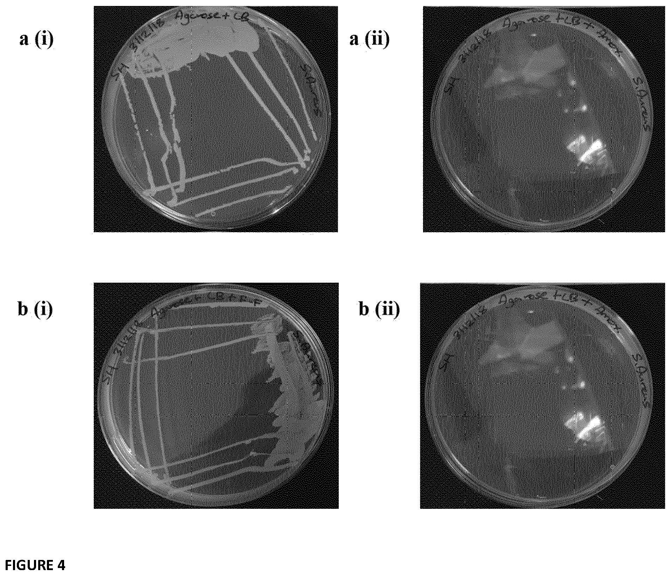

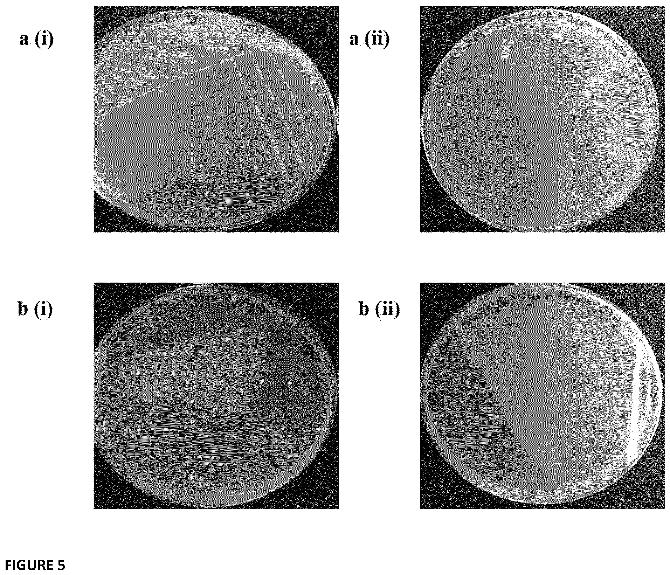

Figure 3 (a) (i) shows growth curves of *S. aureus* on the gel-modified sensors for 150 minutes, until evaporation. It is clear that *S. aureus* pipetted onto the amoxicillin-infused sensor produces an almost identical response to the baseline response, i.e. the situation where bacteria were not present in the 5 ul aliquot mimics inoculation with *S. aureus,* indicating inhibited growth. This data correlates with that obtained for *S. aureus* growth on agarose-plates infused with amoxicillin and incubated and assessed using traditional microbiology techniques (Figures 4 and 5, described below), indicating that the electrochemical technique correlates with the benchmark microbiology work.

Figure 4 presents the results of gel streaking experiments performed using sensitive *S. aureus* featuring gels containing different gel components. All gels contained 1 wt% agarose + LB and some contained FF-C and amoxicillin (8 $\mu$g/ml). The aim of streaking out *S. aureus* on plates was to assess the influence of FF-C and amoxicillin on *S. aureus* growth. From the images, it is clear that *S. aureus* is able to grow in the presence of FF-C (Figure 4 (b) (i)), however, if amoxicillin is present, *S. aureus* growth is completely inhibited regardless of the presence of FF-C.

Figure 5 presents the results of gel streaking experiments performed using sensitive *S. aureus* and methicillin-resistant *S. aureus* (MRSA) featuring gels containing different gel components. All gels contained 1 wt% agarose + LB Broth + FF-C and some contained amoxicillin around the MIC value (8 $\mu$g/ml). The aim of this streaking experiment was to assess the influence of amoxicillin on *S.* aureus/MRSA growth. From the images, it is clear that *S. aureus* is unable to grow in the presence of amoxicillin (Figure 5 (a) (ii)), but grows well in its absence (Figure 5 (a) (i)). However, in the case of MRSA, it displays growth either in the presence or absence of amoxicillin, albeit at a less efficient rate compared to the sensitive strain. This is likely due to the nature of the MRSA frozen stock strain and the method of streaking onto plates. However, some growth is clearly visible in the scenario where amoxicillin is present, unlike in the sensitive *S. aureus* case.

*S. aureus* loaded onto the gel of the invention without amoxicillin gave a different *Z* profile than the profile obtained in the presence of amoxicillin, indicating bacterial growth as a function of time. The baseline data was then subtracted from the two curves in order to clearly visualise the change in *Z* as a function of time, and to pinpoint the bacterial growth period. Figure 3 (a) (ii) shows the result of the baseline subtraction for *S. aureus* growth in the sensor infused with amoxicillin and in the sensor without. Interestingly, *Z* for *S. aureus* on the amoxicillin-laden gel is unchanging between 30 and 100 minutes, whereas, for *S. aureus* on the gel containing no antibiotic, *Z* increases by around 200%, confirming changes to the bacterial concentration on the sensor surface.

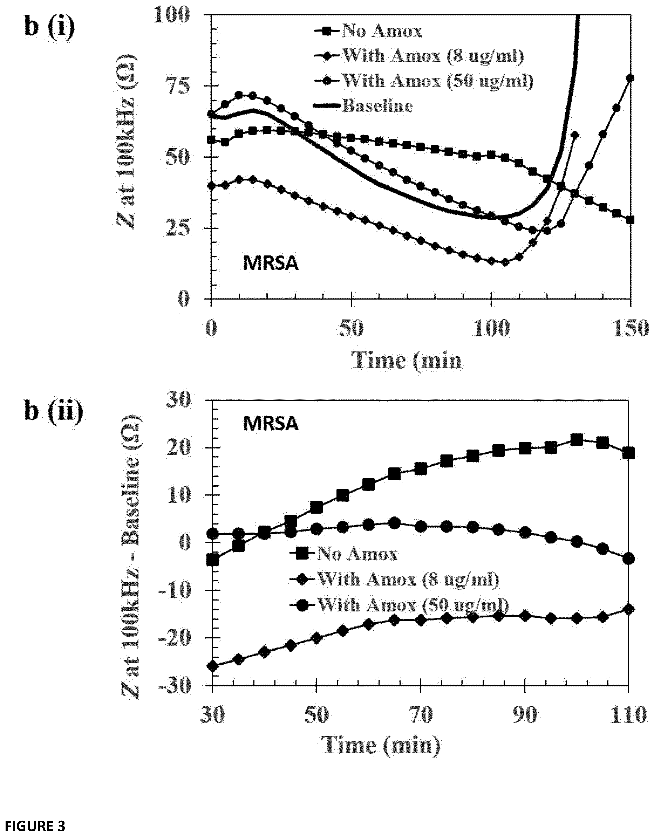

Next, a resistant *S. aureus* strain (MRSA) was investigated in the same manner as for the sensitive *S. aureus* strain. Figure 3 (b) (i) shows growth profiles for MRSA on a sensor modified with amoxicillin at a concentration close to the MIC value (8 $\mu$g/ml) and a gel without amoxicillin present. This time, MRSA behaviour (on the amoxicillin-laden gel) does not imitate the baseline like *S. aureus* was found to, but instead displays a more similar (increasing) *Z* gradient akin to when MRSA growth was measured on the non-antibiotic gel. This is confirmed when the baseline curve is subtracted from the data (Figure 3 (b) (ii)). In this instance, the MRSA baseline subtracted data for each gel shows a steady increase overtime, indicating there is hindered growth occurring in the presence of amoxicillin. Over 30-100 minutes, the change in $Z$ for the gel without amoxicillin is ~ 24 $\Omega$, whereas the change for the 8 $\mu$g/ml amoxicillin-gel is ~ 11 $\Omega$. This is to be expected since antibiotic resistance is not always a "binary situation" (Sabath, L. D. et al., M., 1976. Antimicrobial Agents and Chemotherapy. 9, 962-969) and the antibiotic still has an effect on the resistant strain by retarding growth. Clinically, this manifests as the ability to survive in the presence of the intended therapeutic dose of antibiotic. However, what is clear, is that a difference between the *S. aureus* and MRSA strains could be seen for the same amoxicillin concentration, allowing these two strains to be distinguished within 100 minutes, if not less.

To verify the effect of amoxicillin on the MRSA strain, a growth profile for MRSA on amox infused gel at a concentration significantly greater than the MIC (50 $\mu$g/ml) was produced. At this concentration, amoxicillin should completely inhibit MRSA growth, and the growth profile confirms this is indeed the case since it displays a very similar profile to the baseline measurement (Figure 3 (b) (i)). Once this higher antibiotic concentration measurement is subtracted from the baseline (Figure 3 (b) (ii)), it is evident that the concentration of amoxicillin has a significant effect on the growth of MRSA. Unlike the amoxicillin at 8 $\mu$g/ml which displays hindered growth, there is virtually no change in Z for MRSA on 50 $\mu$g/ml amoxicillin between the same 30 - 100 minutes' time period. The subtracted growth profile is completely flat, indicative of inhibited MRSA growth at this antibiotic concentration.

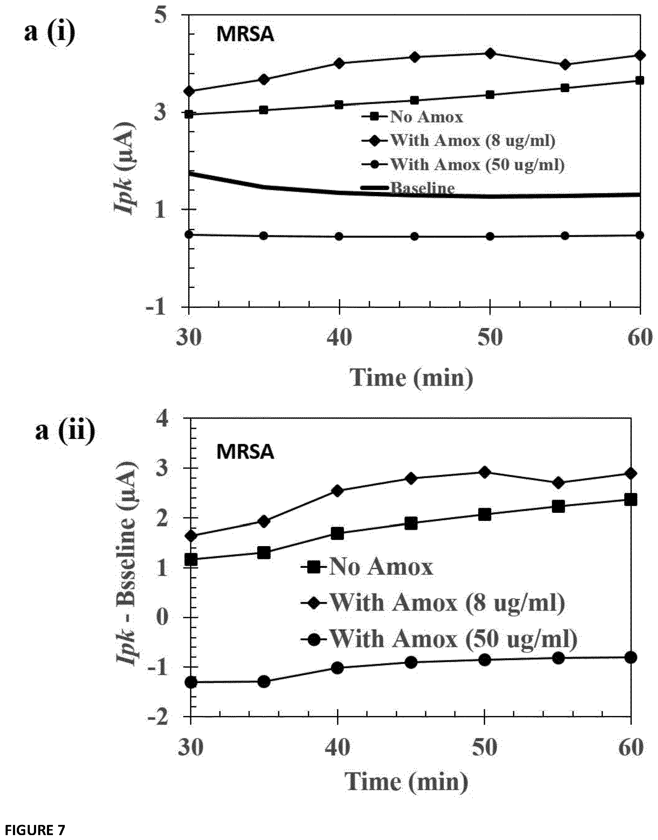

Additionally, growth profiles of MRSA on sensors modified with amoxicillin gels at each antibiotic concentration (8 $\mu$g/ml and 50 $\mu$g/ml) and without antibiotic were produced using the electrochemical technique of differential pulse voltammetry for comparison to Z growth profiles (Figure 7). In this case, the DPV peak current ($I_{pk}$) was plotted as a function of time, and it is clear that $I_{pk}$ displays a similar growth profile to Z. The sensor modified with a gel containing no amoxicillin displays a similar profile (steeper gradient) to the sensor modified with an amoxicillin-laden gel at 8 $\mu$g/ml, whereas for the case of amoxicillin at 50 $\mu$g/ml, once this measurement has been subtracted from the baseline, it is almost completely flat, once again indicative of inhibited MRSA growth at this antibiotic concentration.

Despite the ease of taking DPV measurements, it appears to be more sensitive to changes in gel composition effects over time (for example, drying of the gel), thus Z may be a more sensitive and useful parameter for bacterial growth profiling. However, we note that drying of the gel can be easily prevented by sealing the system and controlling the environment.

### 2.5 Oxacillin modified sensor behaviour

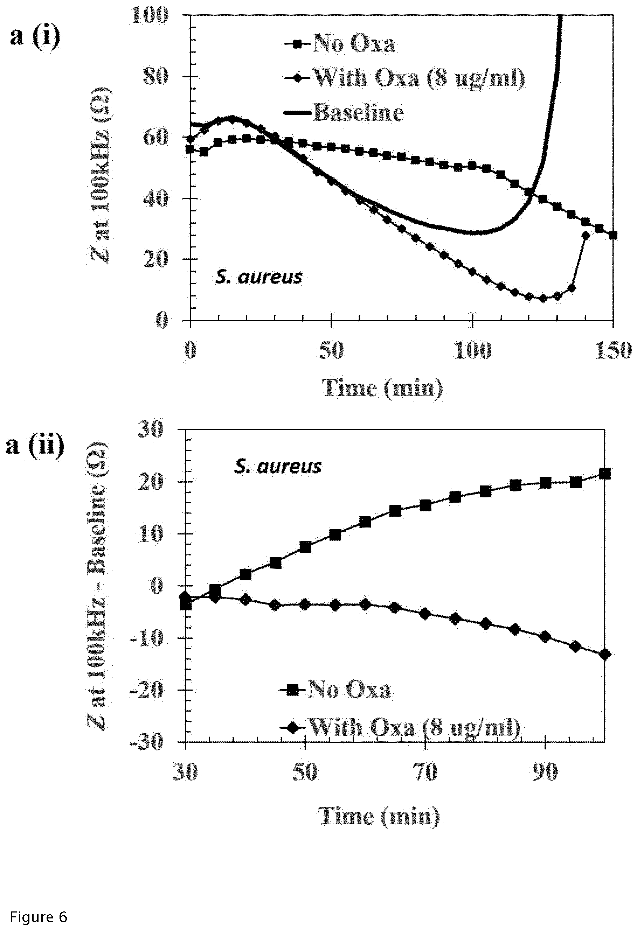

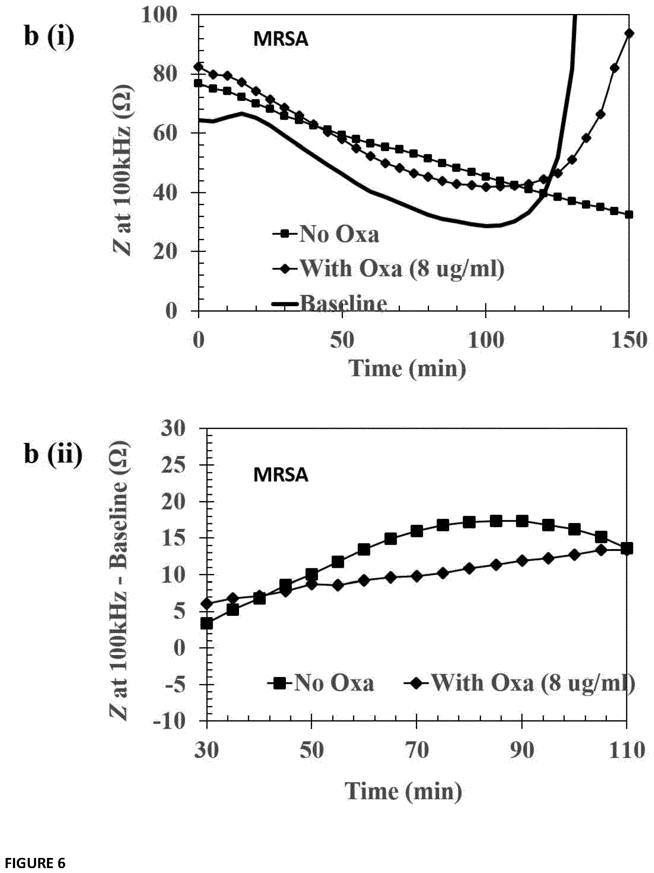

The amoxicillin results were compared with another antibiotic, oxacillin, which is now more commonly used to test for resistance than methicillin. Oxacillin was used to perform electrode growth measurements (Figure 6). Similarly to the growth experiments performed using amoxicillin-laden gels, gels with and without oxacillin were used to investigate *S. aureus* and MRSA growth. Figure 6 (a) (i) presents growth curves of *S. aureus* on an oxacillin-laden gel and a gel containing no antibiotic, as well as the baseline (gel only) curve for comparison. In a similar manner to the results obtained using amoxicillin, *S. aureus* grown on the oxacillin-laden gel produced a very similar $Z$ profile to the baseline measurement where no bacteria were deposited in the 5 $\mu$l aliquot, an indication that oxacillin at 8 $\mu$g/ml is capable of inhibiting *S. aureus* growth. On the other hand, *S. aureus* on the gel with no oxacillin displays a significantly different $Z$ profile, representative of *S. aureus* growth. Figure 6 (a) (ii) displays the growth data with the baseline signal subtracted. In this case, there is a clear distinction between *S. aureus* grown on oxacillin and without. In the case of the gel containing no antibiotic, $Z$ increases by ~ 20 $\Omega$, whereas for the gel with oxacillin, Z remains fairly flat by comparison up to ~ 70 minutes, then a drop-off in Z begins, likely an effect of the gel evaporation process, since for up to 70 minutes in the no antibiotic gel case, there is clear growth in $Z$ from as early as 30 minutes after initial bacteria deposition.

MRSA was also tested on gels infused with and without oxacillin. Figure 6 (b) (i) shows the growth curves produced alongside the baseline data. Unlike *S. aureus,* MRSA growth curves look very similar for gels with and without oxacillin, as previously shown with amoxicillin. There are again subtle differences for the MRSA samples grown on gels containing different levels of oxacillin and these manifest in the gradient of the electrochemical bacterial growth profile. This is to be expected, as explained earlier, drug resistance is a complex phenomenon and in the case of MRSA, the organism is still susceptible to high concentrations of beta-lactam antibiotics. Clinically the resistance manifests as an ability to grow above typical MIC values since these are the conditions under which resistance evolves. The sensor corroborates this; the electrochemical growth profile is most striking in the absence of any antibiotic but shows hindered growth when using sensors modified with antibiotic concentrations close to the MIC. These findings show that the gel/antibiotic modified electrode sensor is able to evaluate bacterial growth and identify drug/dose relationships which retard growth.

### 3. CONCLUSIONS

A low-cost, commercially available, gold screen printed electrode has been used to monitor bacterial growth following modification and using the electrochemical techniques of EIS and DPV. A hydrogel deposit which covered all three electrodes was developed using a common polysaccharide agarose, to benefit from its porous nature, biocompatibility and ability to drop cast. Agarose gels were developed containing different antibiotics and bacterial growth medium to promote or inhibit growth. Growth profiles of both sensitive and resistant *S. Aureus* strains were measured on electrodes modified with gels containing different antibiotic concentrations to perform antibiotic susceptibility testing. The system measured bacterial growth where no antibiotic was present and no growth for sensitive *S. aureus* in the presence of an antibiotic. Furthermore, bacterial growth was measured in the case of low antibiotic concentrations (around the MIC values) for a strain of resistant MRSA.

A clear advantage of this system is the low cost of the sensor and the simplicity of the approach taken. Classical microbiological techniques such as agar plates, growth profiling, MIC assays and susceptibility testing are still in routine use because they work well in the clinical environment. Uptake of PCR based approaches has been slower and this is for a variety of reasons which include: difficulties in persuading health agencies to innovate, PCR reagents can be expensive and also genetic indicators of resistance may be present and identifiable but that does not always convert into the bacteria being resistant (other genetic factors play a role in determining resistance).

The biosensor community has often concentrated on genetic profiling when developing sensors for AMR. However, herein the pragmatic approach of phenotypic testing has been shown to be beneficial. Electrochemical measurements can be recorded on gel-modified electrodes without the need to submerge the sensor into a liquid, there is no need to chemically functionalise the sensor surface with a DNA recognition element (this can be a tremendous source of measurement variation for DNA biosensors) and a wide range of electrochemical parameters can be measured and used to interpret the bacterial growth profile.

The ability to easily modify the gels (antibiotic concentration, nutrient composition, redox agents etc.) in combination with the ability to array a number of electrodes on a single chip is advantageous for use in clinical antibiotic susceptibility testing.

**Development of a rapid, antimicrobial susceptibility test for E. coli based on low-cost, screen-printed electrodes**

**1. Materials and Methods**

Prior to use, commercially available SPEs (as described above) were electrochemically cleaned by cyclic voltammetry (CV) between -0.3 and 1.5 V in 100 mM $H_2SO$ for approximately 10 scans (or until the CV was stable). After cleaning, electrodes were rinsed with DI water and dried using argon.

Gel deposits were nominally produced in 100 ml batches and contained 1 % agarose, 2.5 g Miller LB Broth, 200 mM KCl + 1 mM $Fe[CN]_6^{3-}$ + 1 mM $Fe[CN]_6^{4-}$ (Ferri-ferro Cyanide (FF-C) solution) in DI water. Some gels also contained streptomycin (~ MIC - 4 $\mu$g/ml). All chemicals were purchased from Sigma Aldrich (Dorset, UK). Gels were prepared at room temperature and autoclaved at 121 °C for 15 minutes for sterilisation and to allow the components to mix. Gels harden upon cooling and were therefore stored in a water bath at 48 °C to maintain gel form prior to deposition on the electrodes. Antibiotics were added immediately prior to deposition to avoid inactivation at elevated temperature.

E. coli (ATCC 25922) was streaked out onto plates containing LB media and agar (Sigma Aldrich) from a freshly prepared frozen glycerol stock. Upon growth on LB/Agar plates, single colonies were used to inoculate overnight cultures of Lysogeny Broth (50 ml at 37 °C). Bacteria from the overnight cultures were pipetted directly onto the gel-modified electrodes (5 $\mu$l) at a concentration of ~ 3.5 × 109 CFU/ml, producing a starting E. coli count on the sensor of ~ 1.75 × 107 CFUs. Baseline measurements were performed in a similar manner, except the 5 $\mu$l overnight culture was substituted for 5 $\mu$l of LB medium with no bacteria as a proxy. Streak plates containing gel components contained LB but replaced agar with agarose to match the gel components.

Bacterial electrochemical growth profiles were measured for ~ 5 hours and each experiment involved three SPEs: a baseline measurement (gel only, i.e. no bacteria), gel + antibiotic with bacteria and gel (no antibiotic) with bacteria. Measurements were performed at 37 °C in the test support structure contained within an incubator (Genlab Ltd, Widnes, UK). EIS measurements were performed every 10 min using a measurement script and extracted parameters (e.g. Z at 100 kHz) were plotted as a function of time up to a maximum of ~ 5 h growth post bacterial culture deposition. Each experiment was performed in triplicate.

**1.1 Test support structure design**

The test support structure was developed principally to maintain gel integrity over prolonged time periods allowing for an extended AST measurement window that can capture the growth of microorganisms with longer doubling times (days/weeks) than those on the scale of minutes. The final test support design consisted of three main parts: a stainless steel base plate with slots for up to 6 SPEs (allows multiplexing of 6 SPEs concurrently), a hydrogel enclosure plate (transparent acrylic) and a top lid structure (transparent acrylic) to seal the samples and prevent gel evaporation. The acrylic parts were manufactured using a laser cutter (LPKF ProtoLaser). The base plate and the hydrogel enclosure plate were held together using a combination of countersunk M5 metal screws and rubber O-rings (d=0.8 cm, 2 mm-thick), whereas the lid fitted onto the structure via magnets which were glued securely onto the lid. Before performing electrochemical growth measurements, the structure was tested by depositing 500 μl of water into the chambers and leaving it for several days to ensure an ade-quate seal was achieved, preventing evaporation.

### 1.2 Characterisation

Electrochemical measurements on the SPEs were performed using a three-electrode cell. Measurements were performed using a potentiostat (Palm-Sens PS4, PalmSens, Houten, Netherlands) with associated data analysis software (PSTrace). The SPE was connected to the potentiostat via an edge connector.

Scanning electron microscope (SEM) (TM-1000, Hitachi, Tokyo, Japan) images of the Au working electrode SPE surface were performed by scanning a $150 \times 150$ μm-area at x1.0 k magnification.

Bacterial growth profiles were characterised by electrochemical impedance spectroscopy (EIS) across a frequency range between 100 kHz and 0.1 Hz. Various parameters were examined and Z at 100 kHz and the phase angle at 100 kHz were chosen as the most representative indicators of bacterial growth. The electrochemical parameters were normalised by dividing each data set by their corresponding value at time t = 0 as described in L. Shedden and P. Connolly, World intellectual property organisation, 2010, 26. Independent two-tailed t-tests were then performed to compare the parameters recorded in the presence or absence of antibiotics at each time point (n=3).

## 2. Results and discussion

### 2.1 Sensor overview

A key advantage of the sensor is the phenotypic nature of the gel-modified electrodes which means it is able to detect different types of bacterial infections, and therefore in this case, the common infection E. coli was chosen as the bacteria of interest. Upon deposition of E. coli onto the gel, where no antibiotic is present, the bacteria are able to grow unhindered on the electrode over time. However, when the gel is seeded with antibiotic at a concentration greater than the minimum inhibitory concentration (MIC), the antibiotic causes bacterial growth to be hindered, which is reflected in the electrochemical measurements per-formed in real time.

The measurement setup, consisting of a gel-modified SPE connected to a potentiostat controlled by associated measurement software, can be scaled up to simultaneously monitor several ($\leq 8$) electrodes in real time using a multiplexer format.

A commercially available electrode was used in this study since it is low cost (< £2) and can easily be integrated with the existing measurement setup. The working electrode is 1.6 mm in diameter. An SEM image of the Au WE surface shows that the Au surface is highly irregular and features deep voids and non-homogenous particle sizes.

We have found that our set-up can discern bacterial growth trends directly from 'raw' impedance values at a particular frequency (not requiring a circuit model) ultimately decreasing the instrumentation complexity which facilitates ready deployment at the point of care (POC).

### 2.2 Standardisation experiments

A factor responsible for hydrogel evaporation in previous experimental work was the high temperature incubating conditions employed to promote bacterial growth (37 °C). To minimise the amount of moisture loss, two possible strategies were explored:

1. Increasing the air water vapour content and thus balance the evaporation rate by gel environmental water absorption (hydrogels are highly hygroscopic structures, prone to 'swelling' in humid environments).

2. Enclosing the gel samples within a smaller volume to induce the system to quickly reach saturation (condensation and evaporation rates become equal) and therefore expose the gel to consistent moisture level and effectively cause zero net evaporation.

Implementing the first strategy involved placing a humidifier inside the incubator which would automatically adjust the humidity level based on the desired humidity setting. A baseline (no bacteria) electrochemical measurement was performed simultaneously to investigate the effect of humidity on the resulting electrochemical data. These experiments were only exploratory and therefore were not replicated. The resulting impedance traces obtained following the humidity experiments were reproduced in Fig. 8a. Initial experiments revealed that during normal operation, the humidity level inside the incubator was maintained constant at 20% relative humidity (RH). Prolonged exposure to this humidity level could be problematic not only because of gel evaporation which could destabilise the impedance traces leading to a sharp increase in magnitude, but also for bacterial and fungal growth in general which preferentially takes place in humid conditions.

Increasing the RH level beyond 20% RH would not only slow down the evaporation rate by increasing the air humidity ratio, but also promote water attachment to the polymeric backbone of the hydrogel. However, it was found that electrochemical parameters such as the impedance modulus, which historically was shown to be a useful means of quantifying bacterial growth and metabolic activities, was sensitive to humidity variations (Fig. 8b). At 55% RH ($\pm$ 12% SD), the impedance followed the humidity trace for the entirety of the testing window with only a small time delay in-between (not reproduced). This time delay was likely related to the time required for the RH level to stabilise inside the incubator following humidity adjustment by the humidifying element. When working with a 75% RH level, a measurement where the humidity was much more stable inside the incubator ($\pm$ 4% SD), a linear dependence between the gel electrochemical impedance and environmental humidity was noticed (Fig. 8b).

If left free-standing inside the incubator in a highly humid environment, the gel was found to swell as indicated by the decreasing impedance modulus value at 100 kHz (75% measurement) until it eventually collapsed as shown in Fig. 8d (below image) introducing the additional variable of significant morphological change.

When enclosed within an unsealed support frame to maintain its structural integrity, the hydrogels evaporated in highly humid conditions (80 $\pm$ 7% RH) likely as a result of the evaporation rate surpassing that of water absorption when the effective air exposed area was reduced (rather than a dome, the support gave rise to a 'well' type structure due to wall attachment). These results suggested that, in order to maintain a consistent baseline for bacterial measurements, it is beneficial for the humidity conditions the hydrogel is exposed to during incubation to remain approximately constant throughout the entire testing period, and ideally in the range 90-100% RH. A cheaper and effective alternative to optimal, sensitive humidity control is completely enclosing the hydrogel within a sealed test support to essentially create an atmosphere of zero net evaporation.

Fig. 8c (left) displays the test support structure developed to be able to monitor bacterial growth curves for a longer period of time (several hours) compared to ~ 2 hours previously possible without the test support, due to gel evapo-ration. The stainless steel base and acrylic enclosure and lid are inexpensive and permit aseptic cleaning with 70% ethanol. In addition, acrylic is convenient to manufacture and shows electrochemical inertia. The gel enclosure developed is highlighted in Fig. 8c (right) and shows the way the gel forms in the well. This specific shape results from the hydrophilic interaction of the hydrogel with acrylic through wall attachment. Compared to the previous dome-shaped gel formation, it ensures the full bacterial deposit is contained and tested. An example electrode with the gel in place before baseline data was recorded is displayed in Fig. 8d (top) and upon test support removal after an 8h base-line measurement is shown in Fig. 8d (middle) for comparison. It is clear that the gel maintains its integrity and keeps the distinctive 'well' shape across the entire 8h measurement window.

Using the test support, whilst the gel still evaporates, assuming a perfectly sealed enclosure, condensation balances out that evaporation resulting in zero net evaporation. This in turn enables the establishment of a very flat baseline curve as shown in Fig. 8e. With the support in place, the gel-modified sensor displays an impedance (modulus) (Z) at 100 kHz of ~ 50 $\Omega$, which it maintains for the entire 8 hours under observation. On the contrary, the same measurement without the test support shown in orange, starts around 50 $\Omega$, but then steadily climbs to almost 70 $\Omega$ before fully evaporating within 2 hours. Therefore, the test support brings a better consistency in the deposition of the hydrogel and bacterial sample onto the electrode measuring area, enhancing repeatability of the presented AST technology.

As a result of the ability of the test support to maintain a steady baseline over a significantly longer period of time; subsequent growth profiles with E. coli were performed using the support. The test support provides the ability to monitor organisms with longer doubling times enabling the development of a truly generic AST sensor for any type of bacterial/fungal sample presented.

## 2.3 Bacterial growth profiles

Upon establishment of the test support, validation of the structure with E. coli was carried out and involved depositing a small volume (5 $\mu$l) of an overnight culture of E. coli onto gel-modified and monitoring electro-chemical growth profiles over time. EIS was performed every 10 minutes and various parameters including Z at different frequencies were extracted and plotted over time.

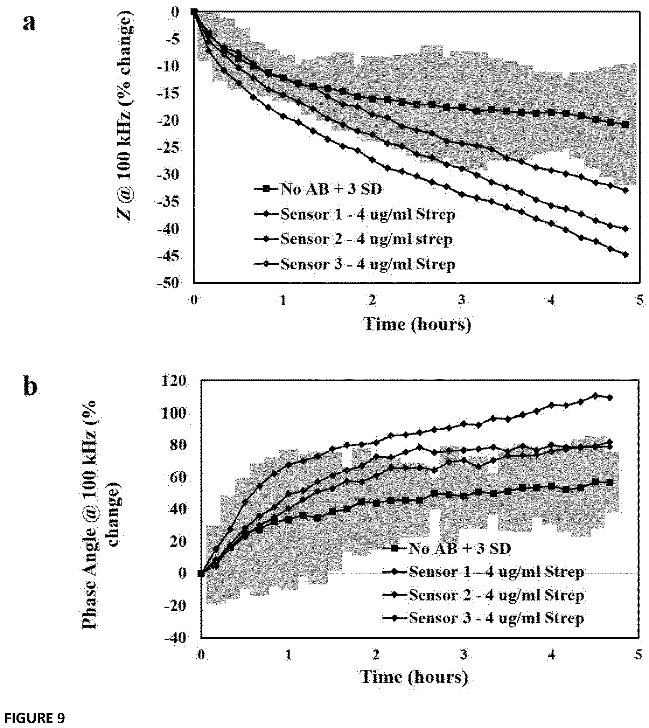

The limit of detection afforded by the device was determined by plotting the 99% confidence zone surrounding the E. coli trace and noting the experimental timeframes required for the antibiotic-infused bacterial traces (n=3) to diverge from that zone (Fig. 9). By averaging these temporal values, it was found that the detection system could successfully discern bacterial growth after only ~ 2.5 hours based on the impedance readout at 100 kHz (a) or ~ 2.1 hours if using the phase angle at 100 kHz (b).

This time-to-result is significantly quicker than the current gold standard for AST of at least 1-2 days, and could be a very useful tool for rapid, low-cost AST testing at the POC. A simplistic form of data analysis (i.e. normalisation and computing the 3 SD zone) was employed to decrease the overall system complexity and make it more amenable for use at the POC. Such a POC device could be used by clinicians to rapidly choose the best antibiotic to treat a particular infection, in timescales much quicker than current methods enable. A test like this one would vastly improve patient health, as well as help avoid the unnecessary prescribing of (typically broad spectrum) antibiotics whilst improving stewardship of our most treasured antimicrobial stocks.

The phenotypic nature of this technology means it is highly versatile and can be used for a wide range of pathogenic organisms (Gram-positive or -negative bacteria) including 'slower' growing organisms such as M. tuberculosis to provide a rapid time-to-result for AST. Furthermore, this method has high-sensitivity enabling detection of E. coli at clinically relevant concentrations, given the UTI bacterial threshold is ≥105 CFU/mL, and typically anywhere up to 108 CFU/ml, and E. coli is the most common uropathogen (see T.K. Price et al., J. Clin. Microbiol., 2016, 54 (5), 1216-1222 and J.K. Brons et al., J. Microbiol. Methods, 2020, 169, 105799). This technology is currently well within the acceptable range for a positive UTI diagnosis. This technology may also find utility in testing clinically relevant fungi such as Candida albicans and Crypto-coccus species.

Fast time-to-result with a simple measurement format at the POC could inform the therapeutic decision independently from resource availability and enhance overall antibiotic stewardship. Moreover, it would allow the monitoring of treatment efficacy for a more personalised, therapeutic approach to improve patient outcomes, maintain antimicrobial efficacy, reduce antimicrobial resistance associated costs and mitigate the spread of AMR. These developments represent a clear step forward towards widespread, low cost and routine antibiotic susceptibility testing which will be critical in the future, where antibiotic prescriptions might not be possible without a confirmatory test due to e.g. government legislation.

**Claims**

1. A method of measuring the antimicrobial susceptibility of microbes comprising:

   (i) contacting a device of a system with at least one antimicrobial or candidate antimicrobial and a sample suspected of comprising or known to comprise microbes wherein the device comprises:

   (a) an electrode system comprising two or more electrodes; and
   (b) a first substance in contact with the two or more electrodes wherein the first substance is in the form of a gel, which is suitable for electrical conductance and which is capable of supporting microbial growth and wherein the first substance comprises a redox mediator selected from a transition metal salt;
   and the system comprises:

   (a) one or more of the devices; and
   (b) a potentiostat,

   wherein the electrodes of the electrode system are electronically connected to the potentiostat;

   (ii) applying an electrical stimulus to the electrodes at least at a first and second time point; and
   (iii) sensing an electrical response from the electrodes at the at least first and second time points;

   wherein a difference in electrical response between the at least first and second time points is indicative of microbial growth.

2. The method of claim 1 wherein the gel is:

   (i) a hydrogel or
   (ii) agarose.

3. The method of any one preceding claim wherein the first substance comprises microbial broth.

4. The method of any one preceding claim wherein the first substance comprises an electrolyte.

5. The method of claim 4 wherein the electrolyte comprises a metal cation selected from any one or a combination of the group consisting of potassium, sodium, magnesium, calcium, zinc and chromium, and a counterion selected from any one or a combination of the group consisting of halide, sulfate, carbonate, bicarbonate, phosphate, nitrate, citrate, gluconate, acetate, oxide, lactate, glubionate, aspartate and picolinate.

6. The method of any one preceding claim wherein the redox mediator is a metal salt, such as $[Fe^{III}(CN)_6]^3$ $7[Fe^{II}(CN)_6]^{4-}$.

7. The method of any one preceding claim wherein the first substance is contacted with the electrodes of the electrode system by:

    (i) deposition; or
    (ii) drop-casting.

8. The method of any one preceding claim wherein the device is sealed.

9. The method of any one preceding claim wherein the electrode system is screen printed.

10. The method of any one preceding claim wherein the at least one antimicrobial is:

    (i) an antibiotic; or
    (ii) any one or a combination selected from the group consisting of amoxicillin and oxacillin.

11. The method of any one preceding claim wherein:

    (i) the electrical stimulus is a potential and the electrical response is a current;
    (ii) the electrical stimulus is a potential comprising either a series of regular voltage pulses or an alternating potential and the electrical response is a current comprises either a direct current or an alternating current; or
    (iii) the electrical stimulus is an alternating potential with a frequency range of 150 Hz to 0.05 Hz; 120 Hz to 0.07 Hz; or 100 Hz to 0.1 Hz; with a waveform amplitude of 20 to 5 mV rms; 15 to 7 mV rms or 10 mV rms and the electrical response is an alternating current.

12. The method of any one preceding claim wherein the method additionally comprises measuring a background electrical response, which comprises:

    (i) contacting the device or system with at least one antimicrobial or candidate antimicrobial;
    (ii) applying an electrical stimulus to the electrodes at least at a first and second time point; and
    (iii) sensing a background electrical response from the electrodes at the at least first and second time points; wherein a difference in background electrical response between the at least first and second time points is background signal;
    and/or

        (i) contacting the device or system with a sample suspected of comprising or known to comprise microbes;
        (ii) applying an electrical stimulus to the electrodes at least at a first and second time point; and
        (iii) sensing a first control electrical response from the electrodes at the at least first and second time points;

    wherein a difference in first control electrical response between the at least first and second time points is first control signal;
    and/or

        (i) contacting the device or system with at least one antimicrobial and microbes known to be susceptible to the at least one antimicrobial;
        (ii) applying an electrical stimulus to the electrodes at least at a first and second time point; and
        (iii) sensing a second control electrical response from the electrodes at the at least first and second time points;

    wherein a difference in second control electrical response between the at least first and second time points is second control signal.

13. A device suitable for measuring antimicrobial susceptibility of microbes, wherein the device is as defined in any one of claims 1 to 6 and comprises at least one antimicrobial or candidate antimicrobial in contact with the first substance wherein the at least one antimicrobial is any one selected from the group consisting of β-lactam antibiotics, macrolides, lincosamides, aminoglycosides, tetracyclines, vancomycin, sulphonamides, fluoroquinolones, chloramphenicol, nitrofurantoin and isoniazid.

14. A system suitable for measuring antimicrobial susceptibility of microbes, wherein the system is as defined in any one of claims 1 to 9 and at least one of the one or more devices comprises at least one antimicrobial or candidate antimicrobial in contact with the first substance wherein the at least one antimicrobial is any one selected from the group consisting of β-lactam antibiotics, macrolides, lincosamides, aminoglycosides, tetracyclines, vancomycin, sulphonamides, fluoroquinolones, chloramphenicol, nitrofurantoin and isoniazid.

15. A kit-of-parts comprising:

(i) at least one device or system as defined in any one of claims 1 to 9; and
(ii) at least one antimicrobial selected from the group consisting of β-lactam antibiotics, macrolides, lincosamides, aminoglycosides, tetracyclines, vancomycin, sulphonamides, fluoroquinolones, chloramphenicol, nitrofurantoin and isoniazid.

**Patentansprüche**

1. Verfahren zum Messen der antimikrobiellen Empfindlichkeit von Mikroben, Folgendes umfassend:

(i) In-Kontakt-Bringen einer Vorrichtung eines Systems mit mindestens einem antimikrobiellen Mittel oder einem antimikrobiellen Kandidatenmittel und einer Probe, von der vermutet wird oder bekannt ist, dass sie Mikroben umfasst, wobei die Vorrichtung Folgendes umfasst:

(a) ein Elektrodensystem, umfassend zwei oder mehr Elektroden; und
(b) eine erste Substanz in Kontakt mit den zwei oder mehr Elektroden, wobei die erste Substanz in der Form eines Gels vorliegt, das für elektrische Leitfähigkeit geeignet ist und in der Lage ist, mikrobielles Wachstum zu unterstützen, und wobei die erste Substanz einen Redoxmediator umfasst, der aus einem Übergangsmetallsalz ausgewählt ist;
und das System Folgendes umfasst:

(a) eine oder mehrere der Vorrichtungen; und
(b) einen Potentiostaten,

wobei die Elektroden des Elektrodensystems elektronisch mit dem Potentiostaten verbunden sind;

(ii) Anlegen eines elektrischen Stimulus an die Elektroden mindestens zu einem ersten und einem zweiten Zeitpunkt; und
(iii) Erfassen einer elektrischen Reaktion von den Elektroden zu dem mindestens ersten und zweiten Zeitpunkt;

wobei eine Differenz in der elektrischen Reaktion zwischen dem mindestens ersten und zweiten Zeitpunkt mikrobielles Wachstum angibt.

2. Verfahren nach Anspruch 1, wobei das Gel Folgendes ist:

(i) ein Hydrogel oder
(ii) Agarose.

3. Verfahren nach einem der vorhergehenden Ansprüche, wobei die erste Substanz eine mikrobielle Brühe umfasst.

4. Verfahren nach einem der vorhergehenden Ansprüche, wobei die erste Substanz einen Elektrolyten umfasst.

5. Verfahren nach Anspruch 4, wobei der Elektrolyt ein Metallkation umfasst, das aus einem oder einer Kombination der Gruppe ausgewählt ist, bestehend aus Kalium, Natrium, Magnesium, Calcium, Zink und Chrom, und ein Gegenion, das aus einem oder einer Kombination der Gruppe ausgewählt ist, bestehend aus Halogenid, Sulfat, Carbonat, Bicarbonat, Phosphat, Nitrat, Citrat, Gluconat, Acetat, Oxid, Lactat, Glubionat, Aspartat und Picolinat.

6. Verfahren nach einem der vorhergehenden Ansprüche, wobei der Redoxmediator ein Metallsalz ist, wie $[Fe^{III}(CN)_6]^{3-}/[Fe^{II}(CN)_6]^{4-}$.

7. Verfahren nach einem der vorhergehenden Ansprüche, wobei die erste Substanz mit den Elektroden des Elektrodensystems in Kontakt gebracht wird durch:

(i) Ablagerung; oder
(ii) Tröpfchengießen.

8. Verfahren nach einem der vorhergehenden Ansprüche, wobei die Vorrichtung versiegelt ist.

9. Verfahren nach einem der vorhergehenden Ansprüche, wobei das Elektrodensystem im Siebdruckverfahren hergestellt wird.

10. Verfahren nach einem der vorhergehenden Ansprüche, wobei das mindestens eine antimikrobielle Mittel Folgendes ist:

(i) ein Antibiotikum; oder
(ii) eines oder eine Kombination, ausgewählt aus der Gruppe, bestehend aus Amoxicillin und Oxacillin.

11. Verfahren nach einem der vorhergehenden Ansprüche, wobei:

(i) der elektrische Stimulus ein Potential ist und die elektrische Reaktion ein Strom ist;
(ii) der elektrische Stimulus ein Potential ist, umfassend entweder eine Reihe regelmäßiger Spannungsimpulse oder ein Wechselpotential, und die elektrische Reaktion ein Strom ist, umfassend entweder einen Gleichstrom oder einen Wechselstrom; oder
(iii) der elektrische Stimulus ein Wechselpotenzial mit einem Frequenzbereich von 150 Hz bis 0,05 Hz; 120 Hz bis 0,07 Hz; oder 100 Hz bis 0,1 Hz ist; mit einer Wellenformamplitude von 20 bis 5 mV rms; 15 bis 7 mV rms oder 10 mV rms und die elektrische Reaktion ein Wechselstrom ist.

12. Verfahren nach einem der vorhergehenden Ansprüche, wobei das Verfahren zusätzlich das Messen einer elektrischen Hintergrundreaktion umfasst, Folgendes umfassend:

(i) In-Kontakt-Bringen der Vorrichtung oder des Systems mit mindestens einem antimikrobiellen Mittel oder einem antimikrobiellen Kandidatenmittel;
(ii) Anlegen eines elektrischen Stimulus an die Elektroden mindestens zu einem ersten und einem zweiten Zeitpunkt; und
(iii) Erfassen einer elektrischen Hintergrundreaktion von den Elektroden zu dem mindestens ersten und zweiten Zeitpunkt;
wobei eine Differenz in der elektrischen Hintergrundreaktion zwischen dem mindestens ersten und zweiten Zeitpunkt ein Hintergrundsignal ist;
und/oder

(i) In-Kontakt-Bringen der Vorrichtung oder des Systems mit einer Probe, von der vermutet wird oder bekannt ist, dass sie Mikroben umfasst,
(ii) Anlegen eines elektrischen Stimulus an die Elektroden mindestens zu einem ersten und einem zweiten Zeitpunkt; und
(iii) Erfassen einer ersten elektrischen Kontrollreaktion von den Elektroden zu dem mindestens ersten und zweiten Zeitpunkt;

wobei eine Differenz in der ersten elektrischen Kontrollreaktion zwischen dem mindestens ersten und zweiten Zeitpunkt ein erstes Kontrollsignal ist;
und/oder

(i) In-Kontakt-Bringen der Vorrichtung oder des Systems mit mindestens einem antimikrobiellen Mittel und Mikroben, von denen bekannt ist, dass sie gegenüber dem mindestens einen antimikrobiellen Mittel empfindlich sind;
(ii) Anlegen eines elektrischen Stimulus an die Elektroden mindestens zu einem ersten und einem zweiten Zeitpunkt; und

(iii) Erfassen einer zweiten elektrischen Kontrollreaktion von den Elektroden zu mindestens dem ersten und zweiten Zeitpunkt;

wobei eine Differenz in der zweiten elektrischen Kontrollreaktion zwischen dem mindestens ersten und zweiten Zeitpunkt ein zweites Kontrollsignal ist.

**13.** Vorrichtung, geeignet zur Messung der antimikrobiellen Empfindlichkeit von Mikroben, wobei die Vorrichtung wie in einem der Ansprüche 1 bis 6 definiert ist und mindestens ein antimikrobielles Mittel oder ein antimikrobielles Kandidatenmittel in Kontakt mit der ersten Substanz umfasst, wobei das mindestens eine antimikrobielle Mittel eines ist, das aus der Gruppe ausgewählt ist, bestehend aus β-Lactam-Antibiotika, Makroliden, Lincosamiden, Aminoglycosiden, Tetracyclinen, Vancomycin, Sulfonamiden, Fluorchinolonen, Chloramphenicol, Nitrofurantoin und Isoniazid.

**14.** System, geeignet zur Messung der antimikrobiellen Empfindlichkeit von Mikroben, wobei das System wie in einem der Ansprüche 1 bis 9 definiert ist und mindestens eine der einen oder der mehreren Vorrichtungen mindestens ein antimikrobielles Mittel oder ein antimikrobielles Kandidatenmittel in Kontakt mit der ersten Substanz umfasst, wobei das mindestens eine antimikrobielle Mittel eines ist, das aus der Gruppe ausgewählt ist, bestehend aus β-Lactam-Antibiotika, Makroliden, Lincosamiden, Aminoglycosiden, Tetracyclinen, Vancomycin, Sulfonamiden, Fluorchinolonen, Chloramphenicol, Nitrofurantoin und Isoniazid.

**15.** Bausatz von Teilen, Folgendes umfassend:

(i) mindestens eine Vorrichtung oder ein System wie in einem der Ansprüche 1 bis 9 definiert; und

(ii) mindestens ein antimikrobielles Mittel, das aus der Gruppe ausgewählt ist, bestehend aus β-Lactam-Antibiotika, Makroliden, Lincosamiden, Aminoglycosiden, Tetracyclinen, Vancomycin, Sulfonamiden, Fluorchinolonen, Chloramphenicol, Nitrofurantoin und Isoniazid.

## Revendications

**1.** Procédé de mesure de la sensibilité antimicrobienne de microbes comprenant :

(i) la mise en contact d'un dispositif d'un système avec au moins un antimicrobien ou un antimicrobien candidat et un échantillon soupçonné de comprendre ou connu comme comprenant des microbes, dans lequel le dispositif comprend :

(a) un système d'électrodes comprenant deux électrodes ou plus ; et
(b) une première substance en contact avec les deux électrodes ou plus dans lequel la première substance se présente sous la forme d'un gel, qui est adéquat pour une conduction électrique et qui est capable de supporter une croissance microbienne et dans lequel la première substance comprend un médiateur redox sélectionné à partir d'un sel de métal de transition ;

et le système comprend :

(a) un ou plusieurs des dispositifs ; et
(b) un potentiostat,

dans lequel les électrodes du système d'électrodes sont connectées électroniquement au potentiostat ;

(ii) l'application d'un stimulus électrique aux électrodes au moins à un premier et un second point temporel ; et
(iii) la détection d'une réponse électrique en provenance des électrodes au niveau des au moins premier et second points temporels ;

dans lequel une différence de réponse électrique entre les au moins premier et second points temporels indique une croissance microbienne.

**2.** Procédé selon la revendication 1, dans lequel le gel est :

(i) un hydrogel ou
(ii) l'agarose.

3. Procédé selon l'une quelconque des revendications précédentes, dans lequel la première substance comprend un bouillon microbien.

4. Procédé selon l'une quelconque des revendications précédentes, dans lequel la première substance comprend un électrolyte.

5. Procédé selon la revendication 4, dans lequel l'électrolyte comprend un cation métallique sélectionné parmi un quelconque ou une combinaison du groupe constitué par le potassium, le sodium, le magnésium, le calcium, le zinc et le chrome, et un contre-ion sélectionné parmi un quelconque ou une combinaison du groupe constitué par un halogénure, un sulfate, un carbonate, un bicarbonate, un phosphate, un nitrate, un citrate, un gluconate, un acétate, un oxyde, un lactate, un glubionate, un aspartate et un picolinate.

6. Procédé selon l'une quelconque des revendications précédentes, dans lequel le médiateur redox est un sel métallique, tel que $[Fe^{III}(CN)_6]^{3-}/[Fe^{II}(CN)_6]^{4-}$.

7. Procédé selon l'une quelconque des revendications précédentes, dans lequel la première substance est mise en contact avec les électrodes du système d'électrodes par :

(i) dépôt ; ou
(ii) coulée de gouttes.

8. Procédé selon l'une quelconque des revendications précédentes, dans lequel le dispositif est scellé.

9. Procédé selon l'une quelconque des revendications précédentes, dans lequel le système d'électrodes est sérigraphié.

10. Procédé selon l'une quelconque des revendications précédentes, dans lequel l'au moins un antimicrobien est :

(i) un antibiotique ; ou
(ii) l'une quelconque ou une combinaison sélectionnée parmi le groupe constitué par l'amoxicilline et l'oxacilline.

11. Procédé selon l'une quelconque des revendications précédentes dans lequel :

(i) le stimulus électrique est un potentiel et la réponse électrique est un courant ;
(ii) le stimulus électrique est un potentiel comprenant soit une série d'impulsions de tension régulières soit un potentiel alternatif et la réponse électrique est un courant comprenant soit un courant continu soit un courant alternatif ; ou
(iii) le stimulus électrique est un potentiel alternatif avec une plage de fréquences de 150 Hz à 0,05 Hz ; de 120 Hz à 0,07 Hz ; ou de 100 Hz à 0,1 Hz ; avec une amplitude de forme d'onde de 20 à 5 mV rms ; de 15 à 7 mV rms ou 10 mV rms et la réponse électrique est un courant alternatif.

12. Procédé selon l'une quelconque des revendications précédentes, dans lequel le procédé comprend en outre la mesure d'une réponse électrique d'arrière-plan, qui comprend :

(i) la mise en contact du dispositif ou du système avec au moins un antimicrobien ou un antimicrobien candidat ;
(ii) l'application d'un stimulus électrique aux électrodes au moins à un premier et un second point temporel ; et
(iii) la détection d'une réponse électrique d'arrière-plan en provenance des électrodes au niveau des au moins premier et second points temporels ;
dans lequel une différence de la réponse électrique d'arrière-plan entre les au moins premier et second points temporels est un signal d'arrière-plan ;
et/ou

(i) la mise en contact du dispositif ou du système avec un échantillon soupçonné de comprendre ou connu comme comprenant des microbes ;
(ii) l'application d'un stimulus électrique aux électrodes au moins à un premier et un second point temporel ; et

(iii) la détection d'une première réponse électrique de contrôle en provenance des électrodes au niveau des au moins premier et second points temporels ;

dans lequel une différence de la première réponse électrique de contrôle entre les au moins premier et second points temporels est un premier signal de contrôle ;
et/ou

(i) la mise en contact du dispositif ou du système avec au moins un antimicrobien et des microbes connus comme étant sensibles à l'au moins un antimicrobien ;
(ii) l'application d'un stimulus électrique aux électrodes au moins à un premier et un second point temporel ; et
(iii) la détection d'une seconde réponse électrique de contrôle en provenance des électrodes au niveau des au moins premier et second points temporels ;

dans lequel une différence de la seconde réponse électrique de contrôle entre les au moins premier et second points temporels est un second signal de contrôle.

13. Dispositif adéquat pour mesurer la sensibilité antimicrobienne de microbes, dans lequel le dispositif est tel que défini dans l'une quelconque des revendications 1 à 6 et comprend au moins un antimicrobien ou un antimicrobien candidat en contact avec la première substance, dans lequel l'au moins un antimicrobien est l'un quelconque sélectionné parmi le groupe constitué par les antibiotiques au β-lactame, les macrolides, les lincosamides, les aminoglycosides, les tétracyclines, la vancomycine, les sulfonamides, les fluoroquinolones, le chloramphénicol, la nitrofurantoïne et l'isoniazide.

14. Système adéquat pour mesurer la sensibilité antimicrobienne de microbes, dans lequel le système est tel que défini dans l'une quelconque des revendications 1 à 9 et au moins l'un des un ou plusieurs dispositifs comprend au moins un antimicrobien ou un antimicrobien candidat en contact avec la première substance, dans lequel l'au moins un antimicrobien est l'un quelconque sélectionné parmi le groupe constitué par les antibiotiques au β-lactame, les macrolides, les lincosamides, les aminoglycosides, les tétracyclines, la vancomycine, les sulfonamides, les fluoroquinolones, le chloramphénicol, la nitrofurantoïne et l'isoniazide.

15. Kit de parties comprenant :

(i) au moins un dispositif ou un système tel que défini dans l'une quelconque des revendications 1 à 9 ; et
(ii) au moins un antimicrobien sélectionné parmi le groupe constitué par les antibiotiques au β-lactame, les macrolides, les lincosamides, les aminoglycosides, les tétracyclines, la vancomycine, les sulfonamides, les fluoroquinolones, le chloramphénicol, la nitrofurantoïne et l'isoniazide.

Figure 1

c (i)

c (ii)

Figure 1

d (i)

d (ii)

FIGURE 1

**a**

Figure 2

**b**

**c**

Figure 2

FIGURE 2

38

Figure 3

**FIGURE 3**

a (i)

a (ii)

b (i)

b (ii)

**FIGURE 4**

a (i)

a (ii)

b (i)

b (ii)

FIGURE 5

Figure 6

b (i)

b (ii)

FIGURE 6

FIGURE 7

Figure 8

**c**

- SPE electrodes
- Clear acrylic top/lid
- Hydrogel enclosure
- Sealing magnets
- Base plate + 6 SPE slots

Bacteria culture
Enclosure
Enclosure
Gel deposit

**d**

**e**

FIGURE 8

FIGURE 9

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **FOOD AND AGRICULTURE ORGANIZATION OF THE UNITED NATIONS.** *Antimicrobial Resistance Policy Review and Development Framework,* 2018
- *World Health Organization Global Action Plan on Antimicrobial Resistance,* 2015
- **TODD, A. ; WORSLEY, A. ; ANDERSON, R. J. ; GROUNDWATER, P. W.** *The Pharmaceutical Journal,* 2009, vol. 283, 359-360
- **DAVIES, J. ; DAVIES, D.** *Microbio and Mol. Bio. Rev.,* 2010, vol. 74, 417-433
- **MCEWEN, S. A. ; COLLIGNON, P. J.** *Microbiol Spectr.,* 2018, 6
- **DEPARTMENT OF HEALTH & SOCIAL CARE.** *The UK's vision for AMR by 2040 and five-year national action plan,* 2019
- **GILLIGAN, P. H.** The Prokaryotes, Human Microbiology. Springer Reference, 2013
- **CLARRIDGE, J. E.** *Diseases. Clin. Microbiol. Rev.,* 2004, vol. 17, 840-862
- **NIE, Z. ; NIJHUIS, C. A. ; GONG, J. ; CHEN, X. ; KUMACHEV, A. ; MARTINEZ, A. W. ; NAROVLYANSKY, M. ; WHITESIDES, G. M.** *Lab Chip.,* 2010, vol. 10, 477-483
- **WANG, W. ; HUANG, H. Y. ; CHEN, S. C. ; HO, K. C. ; LIN, C. Y. ; CHOU, T. C. ; HU, C. H. ; WANG, W. F. ; WU, C. F. ; LUO, C. H.** *Sensors,* 2011, vol. 11, 8593-8610
- **WARD, A. C. ; HANNAH, A. J. ; KENDRICK, S. L. ; TUCKER, N. P. ; MACGREGOR, G. ; CONNOLLY, P.** *Biosensors and Bioelectronics,* 2018, vol. 110, 65-70
- **BROSEL-OLIU, S. ; MERGEL, O. ; URIA, N. ; ABRAMOVA, N. ; VAN RIJN, P. ; BRATOV, A.** *Lab. Chip,* 2019, vol. 19, 1436-1447
- **SETTU, K. ; LIU, J. T. ; CHEN, C. J. ; TSAI, J. Z. ; CHANG, S. J.** *Conf. Proc. IEEE Eng. Med. Biol. Soc.,* 2013, vol. 2013, 1712-1715
- **WARD, A. C. ; CONNOLLY, P. ; TUCKER, N. P.** *PLOS One,* 2014, vol. 9, 91732
- **BATCHELOR-MCAULEY, C. ; KATELHON, E. ; BARNES, E. O. ; COMPTON, R. G. ; LABORDA, E. ; MOLINA, A.** *ChemistryOpen,* 2015, vol. 4, 224-260
- **JIANG, D.,GE, P., WANG, L., JIANG, H., YANG, M., YUAN, L., GE, Q., FANG, W., JU, X.** *Biosensors and Bioelectronics,* 2019, vol. 130, 299-306

- **RUSSELL, C. ; WARD, A. C. ; VEZZA, V. ; HOSKISSON, P. ; ALCORN, D. ; STEENSON, D. P. ; CORRIGAN, D. K.** *Biosensors and Bioelectronics,* 2019, vol. 126, 806-814
- Monitoring Pseudomonas aeruginosa in culture plates using embedded electrochemical sensors. **WEBSTER et al.** 40th Annual NorthEast Bioengineering Conference (NEBEC). IEEE, 2014, 1-2
- **BADALYAN et al.** Novel sensor platform for rapid detection and quantification of coliforms on food contact surfaces. *Journal of Microbiological Methods,* 2018, vol. 153, 74-83
- **BESANT ; SARGENT ; KELLEY.** Rapid electrochemical phenotypic profiling of antibiotic-resistant bacteria. *Lab Chip,* 2015, vol. 15, 2799-2807
- **TAVAKOLI, J. ; TANG, Y.** *Mater. Sci. Eng. C. Mater. Biol. Appl.,* 2017, vol. 77, 318-325
- **HAN, L. ; LU, X. ; WANG, M. ; GAN, D. ; DENG, W. ; WANG, K. ; FANG, L. ; LIU, K. ; CHAN, C. W. ; TANG, Y.** *Small,* 2017, 13
- **CHEN, S. ; DUAN, J. ; TANG, Y. ; ZHANG QIAO, S.** *Chemistry,* 2013, vol. 19, 7118-7124
- **MICHALEK, J. ; HOBZOVA, R. ; PRADNY, M. ; DUSKOVA, M.** Biomedical Applications of Hydrogels Handbook. 2010, 303-316
- *Science Direct,* 2019
- **JEPPSSON, J. O. ; LAURELL, C. B. ; FRANZEN, B.** *Clin. Chem.,* 1979, vol. 25, 629-638
- **SABATH, L. D. ; GARNER, C. ; WILCOX, C. ; FINLAND, M.** *Antimicrobial Agents and Chemotherapy,* 1976, vol. 9, 962-969
- **J. OVERMANN et al.** *Annu. Rev. Microbiol.,* vol. 71, 711-730
- **TAN, C. ; NASIR, M. Z. M. ; AMBROSI, A. ; PUMERA, M.** *Anal. Chem.,* 2017, vol. 89, 8995-9001
- **HAYAT, A. ; MARTY, J. L.** *Sensors,* 2014, vol. 14, 10432-10453
- **TAYLOR, T. A. ; UNAKAL, C. G.** StatPearls. Treasure Island Publishing, 2019
- **TONG, S. Y. C. ; DAVID, J. S. ; EICHENBERGER, E. ; HOLLAND, T. L. ; FOWLER JR, V. G.** *Clin. Microbiol. Rev.,* 2015, vol. 28, 603-661
- **HARKINS, C. P. ; PICHON, B. ; DOUMITH, M. ; PARKHILL, J. ; WESTH, H. ; TOMASZ, A. ; DE LENCASTRE, H. ; BENTLEY, S. D. ; KEARNS, A. M. ; HOLDEN, M. T. G.** *Genome. Biol.,* 2017, vol. 18, 130

- **COLL, F. ; HARRISON et al.** *Sci. Transl. Med.,* 2017, vol. 9, 1-19
- **BARRY, A. L. et al.** *J. Clin. Microbiol.,* 1992, vol. 30, 585-589
- **KURALAY, F. et al.** *Talanta,* 2015, vol. 85, 1330-1337
- **NDIAYE, A. L. et al.** *Biosensors,* 2016, vol. 6, 46
- **SABATH, L. D. et al.** *Antimicrobial Agents and Chemotherapy,* 1976, vol. 9, 962-969
- **L. SHEDDEN ; P. CONNOLLY.** *World intellectual property organisation,* 2010, 26
- **T.K. PRICE et al.** *J. Clin. Microbiol.,* 2016, vol. 54 (5), 1216-1222
- **J.K. BRONS et al.** *J. Microbiol. Methods,* 2020, vol. 169, 105799